# EUROPEAN PATENT APPLICATION

(11) **EP 4 459 623 A1**
(43) Date of publication of application: **06.11.2024**
(21) Application number: 23740304.3
(22) Date of filing: 12.01.2023
(51) Int. Cl.: G16B 30/00, G16B 20/20, G16H 50/20

(54) **INFORMATION PROCESSING DEVICE, INFORMATION PROCESSING METHOD, AND PROGRAM**

(30) Priority: 13.01.2022 JP 2022003784
(71) Applicant: The University of Tokyo, Bunkyo-ku, Tokyo 113-8654 (JP)
(72) Inventor: YOKOYAMA Kazuaki, Tokyo 113-8654 (JP)
(74) Representative: Appleyard Lees IP LLP
(86) International application number: PCT/JP2023/000620
(87) International publication number: WO 2023/136296

(57) **Abstract**

The purpose of the present invention is to improve the efficiency and convenience of analyzing the degree of likelihood that a mutation is one that affects the development or progression of a disease. A common filter unit 53 of an analysis device 1 for selecting a cancer driver mutation in a subject classifies, on the basis of a first classification criteria, each of a plurality of sequence mutations identified by sequencing nucleic acids included in the subject into a rank from among rank MYC1 for which the likelihood of being selected as a target sequence mutation is the highest and ranks MYC2 to MYC4 for which the above likelihood is lower than that for MYC1. A parameter-setting reception unit 542 sets, as a second classification criteria, a classification criteria of having been registered in a database or a list, the second classification criteria being different from the first classification criteria for carrying out classification into rank MYC1. A seed gene filter 541 reclassifies, into rank MYC1, a sequence mutation that satisfies the second classification criteria from among the sequence mutations classified into ranks MYC2 to MYC4. The problem is thereby solved.

## Description

### TECHNICAL FIELD

The present invention relates to an information processing device, an information processing method, and a program.

### BACKGROUND ART

It has been widely known that diseases may occur due to mutations in the base sequences contained in the genetic information of somatic cells. Recently, information regarding various somatic mutations and their association with specific diseases has been collected and recorded in databases, which are widely used (see Non-Patent Document 1).

With the recent advancements in comprehensive base sequence analysis technologies (such as next-generation sequencers), the number of mutations detected in a single analysis has become enormous, ranging from hundreds to millions per sample. Manually interpreting the results of each mutation is inefficient and impractical. Therefore, there is a demand for devices that assist human interpretation of analysis results.

### Citation List

### Non-Patent Document

Non-Patent Document 1: COSMIC Release v94 is live!, [online], March 28, 2021, [search conducted on October 8, 2021], Internet <URL: https://cosmic-blog . sanger.ac.uk/Release-v94/>

### DISCLOSURE OF THE INVENTION

### Problems to be Solved by the Invention

However, conventional databases merely recorded the mutations that occurred in the cases. Consequently, analyzing base sequence variations using the database only allowed for determining whether the databased mutations were present but did not provide a definitive judgment on whether the mutations directly influenced the development or progression of diseases such as cancer (e.g., driver mutations for cancer). In other words, interpreting the analysis results of mutation analysis was challenging due to the numerous factors to consider in determining whether a mutation was a driver mutation. The Applicant has previously filed a patent application for a technology achieving an analyzer that provides the degree of likelihood that a mutation affects the onset or progression of diseases (see International Application No. PCT/JP2020/037499). However, beyond such analyzers, there remains a demand for further improving the efficiency and convenience of analyzing the degree of likelihood that a mutation affects the onset or progression of diseases.

The present invention has been made in view of such circumstances, and an object of the present invention is to improve the efficiency and convenience of analyzing the degree of likelihood that a mutation affects the onset or progression of diseases.

### Means for Solving the Problems

In order to achieve the above object, one aspect of the information processing device according to the present invention is an information processing device that selects target sequence variations that are present in a subject and that pose a risk of harm, in which the device includes:
a first filterer that classifies each of the plurality of sequence variations identified by sequencing the nucleic acids contained in the subject, based on a first classification criterion, into either a high category that categorizes sequence variations with the highest likelihood of being selected as the target sequence variations or at least one lower category with a lower likelihood;
a classification criterion setter that sets a classification criterion of being registered in a database or list, as a second classification criterion different from the first classification criterion for classification into the high category; and
a second filterer that reclassifies the sequence variations, which have been classified into the lower category by the first filterer but satisfy the second classification criterion, into the high category.

Each of the information processing methods and programs according to one aspect of the present invention corresponds to the method and program of the information processing device according to one aspect of the present invention.

### Effects of the Invention

According to the present invention, it is possible to improve the efficiency and convenience of analyzing the degree of likelihood that a mutation affects the onset or progression of diseases.

### BRIEF DESCRIPTION OF THE DRAWINGS

FIG. 1 is a block diagram illustrating an example of the hardware configuration of an analyzer according to one embodiment of the information processing device of the present invention;
FIG. 2 is a block diagram illustrating an example of the functional configuration of the analyzer of FIG. 1;
FIG. 3 illustrates an example of the configuration of the mutant base sequence information received by the analyzer of FIG. 2;
FIG. 4 illustrates an example of the configuration of the analysis result information outputted by the analyzer of FIG. 2;
FIG. 5 is a block diagram illustrating an example of the detailed functional configuration of the common filter unit of the analyzer of FIG. 2;
FIG. 6 is a block diagram illustrating an example of the detailed functional configuration of the seed gene filter unit of the analyzer of FIG. 2;
FIG. 7 is a schematic diagram illustrating the significance of adopting the seed gene filter unit in the analyzer of FIG. 2;
FIG. 8 illustrates an example of a screen for parameter input to the seed gene filter unit and the rescue filter of the analyzer of FIG. 2;
FIG. 9 is a flowchart illustrating an example of the analysis processing flow in the analyzer with the functional configuration of FIG. 6;
FIG. 10 is a flowchart illustrating the details of the common filter processing flow in the analysis processing of FIG. 9;
FIG. 11 is a flowchart illustrating the details of the seed gene filter processing flow in the analysis processing of FIG. 9;
FIG. 12 is a flowchart illustrating the details of the rescue filter processing flow in the analysis processing of FIG. 9;
FIG. 13 is a block diagram illustrating another example of the detailed functional configuration of the common filter unit of the analyzer of FIG. 2, different from the example in FIG. 5;
FIG. 14 is a flowchart illustrating the details of the common filter processing flow by the common filter unit with the functional configuration of FIG. 13;
FIG. 15 is a diagram illustrating an example of inference using an AI model generated or updated by machine learning in the rescue filter processing of FIG. 12; and
FIG. 16 is a diagram illustrating an example of updating the AI model generated or updated by machine learning in the rescue filter processing of FIG. 12.

### PREFERRED MODE FOR CARRYING OUT THE INVENTION

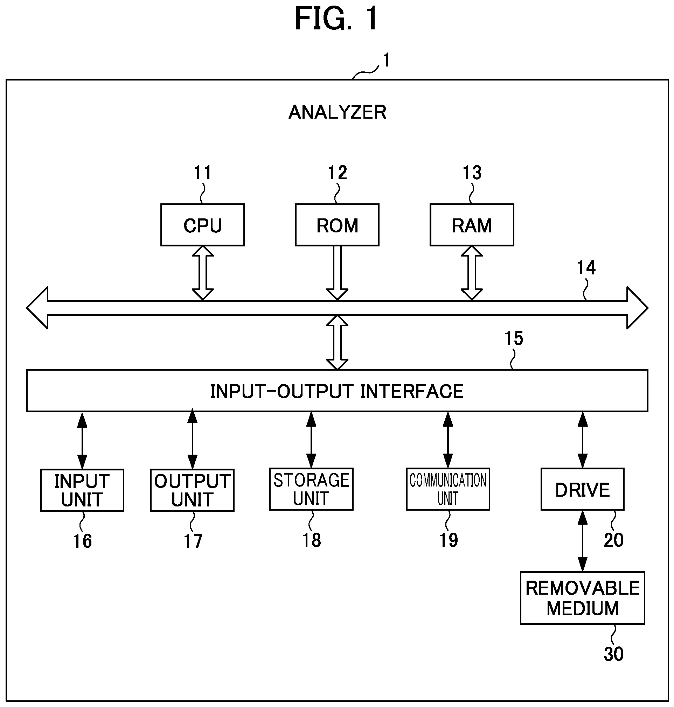

Hereinafter, embodiments of the present invention will be described with reference to the drawings. FIG. 1 is a block diagram illustrating an example of the hardware configuration of an analyzer according to an embodiment of the information processing device of the present invention.

An analyzer 1 receives: sample identification information for identifying an individual as an analysis target and a sample obtained from the individual; and mutant base sequence information representing the mutation status (sequence variation) including the mutation sites and the details of mutation of the base sequence extracted through sequence alignment from the genetic information of the sample. The mutation status (sequence variation) may be a single base mutation or a structural variant such as a chromosomal translocation involving a plurality of genes. Specifically, the mutation sites and the details of mutation include information indicating the location of the mutation (e.g., the number of bases from one end of the chromosome as compared with the reference genome information) and information indicating which base has mutated from the expected base. For NGS analysis, reference genome information such as GRCh38 (hg38) or GRCh37 (hg19) can be used for human.

The analyzer 1 classifies each mutation status (sequence variation) included in the received mutant base sequence information into provisional ranks, based on whether each mutation status (sequence variation) represented by the received mutant base sequence information satisfies a plurality of predefined classification criteria. Then, the analyzer 1 reclassifies each mutation status (sequence variation) by changing the set provisional ranks, based on whether the degree of likelihood of pathogenicity of each mutation status (sequence variation) satisfies the classification criteria different from the aforementioned classification criteria, based on the provisional ranks classified for each mutation status (sequence variation). This operation of the analyzer 1 will be described in detail later.

The analyzer 1 includes a CPU (Central Processing Unit) 11, a ROM (Read Only Memory) 12, a RAM (Random Access Memory) 13, a bus 14, an input-output interface 15, an input unit 16, an output unit 17, a storage unit 18, a communication unit 19, and a drive 20.

The CPU 11 executes various processing in accordance with programs recorded in the ROM 12 or programs loaded from the storage unit 18 into the RAM 13. The RAM 13 also stores data necessary for the CPU 11 to execute various processing as needed.

The CPU 11, the ROM 12, and the RAM 13 are interconnected via the bus 14. The input-output interface 15 is also connected to the bus 14. The input-output interface 15 is connected to the input unit 16, the output unit 17, the storage unit 18, the communication unit 19, and the drive 20.

The input unit 16 is configured with a keyboard or similar device for inputting various information. The output unit 17, including a display such as an LCD or speakers, outputs various information as images or sounds. The storage unit 18, configured with DRAM (Dynamic Random Access Memory) or similar, stores various data. The communication unit 19 communicates with other devices (e.g., an information processing device of a terminal for viewing analysis results, not illustrated) via a network N, including the internet.

The drive 20 can appropriately mount a removable medium 31, such as a magnetic disk, optical disk, magneto-optical disk, or semiconductor memory. Programs read from the removable medium 31 by the drive 20 are installed in the storage unit 18 as necessary. The removable medium 31 can also store various data stored in the storage unit 18, similar to the storage unit 18.

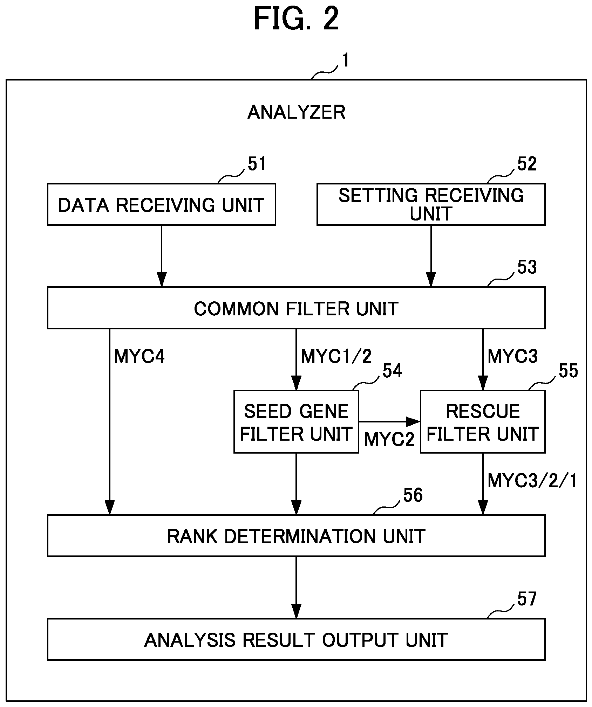

The cooperation of the various hardware and software components of the analyzer 1 illustrated in FIG. 1 enables the execution of various processing. The functional configuration for executing these processing in the analyzer 1 of the present embodiment will be described below with reference to FIG. 2. FIG. 2 is a block diagram illustrating an example of the functional configuration of the analyzer illustrated in FIG. 1.

As illustrated in FIG. 2, a data receiving unit 51, a setting receiving unit 52, a common filter unit 53, a seed gene filter unit 54, a rescue filter unit 55, a rank determination unit 56, and an analysis result output unit 57 function in the analyzer 1.

The data receiving unit 51 receives mutant base sequence information representing the mutation status (sequence variation) of the base sequence, which is extracted through sequence alignment from the genetic information of the sample as an analysis target.

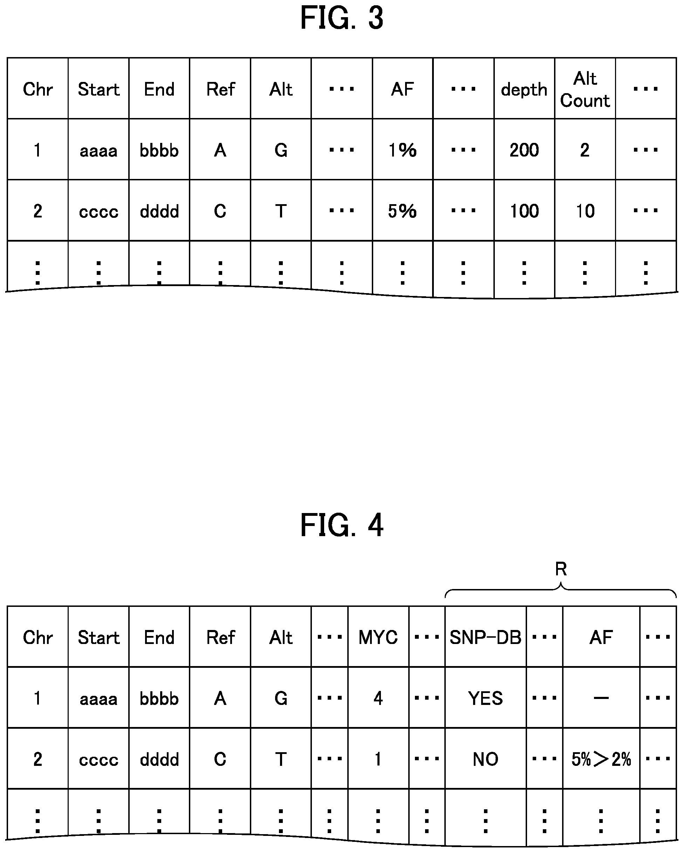

FIG. 3 illustrates an example of the configuration of the mutant base sequence information received by the analyzer illustrated in FIG. 2. As illustrated in FIG. 3, the mutant base sequence information at least associates each mutation status (sequence variation) (each row in FIG. 3) with the number of the chromosome (Chr) in which the base sequence of the mutation status (sequence variation) has been identified, the start location (Start), the end location (End), the reference base sequence (Ref), the extracted mutated base sequence (hereinafter referred to as "sequence variation") (Alt), and the proportion of the sequence variations (allele frequency: AF). In this example of mutant base sequence information, each mutation status (sequence variation) (each row in FIG. 3) is further associated with quality-related indicators such as the depth and the count number of the mutation status (sequence variation) (AltCount), in addition to the aforementioned information. The length of the base sequence may be "1" (in this case, the base sequence information represents one of A, T, C, or G).

The mutant base sequence information may also include information related to the individual's case (such as the disease name, treatment history, and tumor percentage).

The data receiving unit 51 may receive mutant base sequence information (time-series information) extracted from the same individual at timings (which may be a plurality of timings) different from the timing when the mutant base sequence information as an analysis target was extracted. In this case, the data receiving unit 51 receives an input specifying the mutant base sequence information at the time designated as the analysis target.

The setting receiving unit 52 receives analysis settings. The settings include, for example, settings of which filters to use, and settings of parameters, in the common filter unit 53. For easier understanding of the present embodiment, the settings for the seed gene filter unit 54 and the rescue filter unit 55 are performed in the seed gene filter unit 54 and the rescue filter unit 55, respectively; however, the settings may also be performed in the setting receiving unit 52. Specific examples of the settings in the common filter unit 53 will be described along with the configuration of the common filter unit 53.

In the present embodiment, the common filter unit 53 performs the primary evaluation of the likelihood of pathogenicity (e.g., the likelihood of a driver mutation), based on various information affecting the interpretation of mutation analysis results. The evaluation result is represented by one of four ranks, MYC1 to MYC4, described below. The term "primary evaluation" is used herein because, in this example, the evaluation by the common filter unit 53 is followed by reevaluation (re-ranking) by the seed gene filter unit 54 and the rescue filter unit 55. Here, the information affecting the interpretation includes (1) ancillary information of the mutation obtained during analysis, and (2) information related to the mutation recorded in literature and databases. The ancillary information of the mutation obtained during analysis (1) includes (a) detection accuracy and reliability information (the probability that the mutation is not a detection error), (b) allele frequency of the mutation (an indicator related to the proportion of the cell population with the same mutation), and (c) time-series information, i.e., whether the mutation has been repeatedly detected in samples from the same case at different times.

The information related to the mutation recorded in literature and databases (2) includes information indicating whether the mutation is described as a driver mutation of a disease (or the frequency of such descriptions). When registrations are also found in Single Nucleotide Polymorphism (SNP) databases, literature and databases may contain information on how frequently the mutation allele has been reported as an SNP in that racial group. Additionally, literature and databases may contain information predicting whether the mutation affects the three-dimensional structure or function of the protein encoded by the mutation, such as being involved in the pathogenesis of cancer, as demonstrated by experiments or predictions.

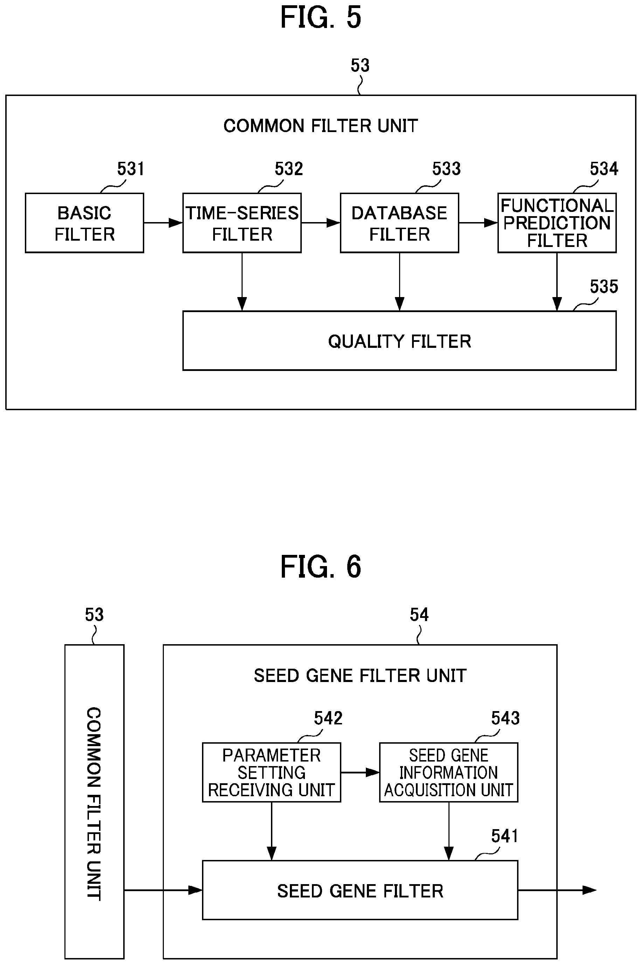

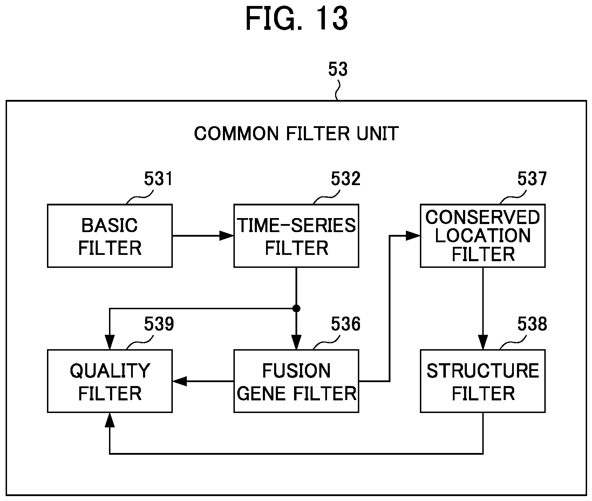

The common filter unit 53 performs a primary evaluation by classifying the plurality of mutation statuses (sequence variations) (when time-series information is received, the mutation status (sequence variation) included in the mutant base sequence information specified as an analysis target, hereinafter referred to as "mutation status (sequence variation) as an analysis target") received by the data receiving unit 51, into any one of the ranks MYC1 to MYC4, based on each of the plurality of predetermined classification criteria. Detailed examples of the configuration of the common filter unit 53 will be described later with reference to FIGS. 5 and 13.

Here, the ranks MYC1 and MYC2 indicate that the sequence variation is evaluated as highly likely to be a driver mutation, i.e., a candidate driver mutation. The rank MYC1 indicates a higher likelihood of a true driver mutation than the rank MYC2. The rank MYC3 indicates that the sequence variation is evaluated as unlikely to be a driver mutation (and thus not treated as a candidate driver mutation). In other words, the rank MYC3 indicates that the sequence variation is evaluated as a non-harmful mutation. The rank MYC4 indicates that the sequence variation is evaluated as almost certainly not a driver mutation, i.e., a known SNP or a mutation in an error-prone region.

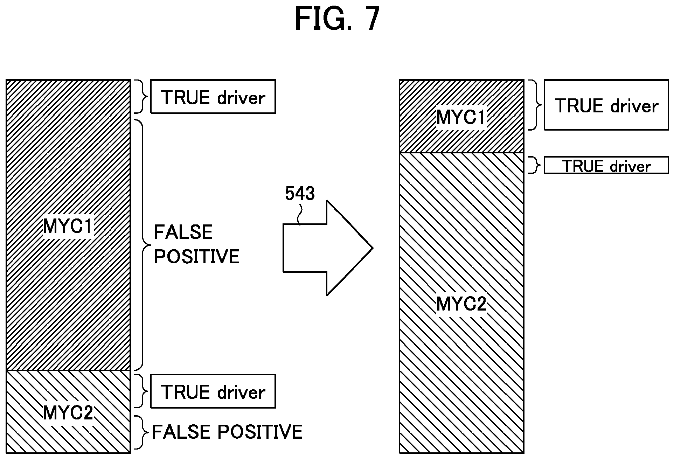

The reason for classifying the plurality of mutation statuses (sequence variations) received by the data receiving unit 51 into the four ranks, MYC1 to MYC4, is as follows. Namely, the number of mutation statuses (sequence variations) is too large (e.g., tens of thousands to hundreds of millions) for users such as specialists to efficiently find true driver mutations. Specifically, the reason is to enable users such as specialists to focus on the mutation statuses (sequence variations) classified into the rank MYC1 or MYC2 to find true driver mutations efficiently. As mentioned above, since the mutation statuses (sequence variations) of the rank MYC1 are defined as highly likely to be true driver mutations, it is more efficient for users such as specialists to focus particularly on the mutation statuses (sequence variations) of the rank MYC1. However, as detailed later, the common filter unit 53 is configured with filters using the classification criteria common to all cancers and genetic diseases. Therefore, in the primary evaluation by the common filter unit 53, depending on the type of carcinoma or genetic disease, true driver mutations may be abundant in the sequence variations of the rank MYC2, or conversely, false positives may be abundant in the sequence variations of the rank MYC1. Details on this point will be described later with reference to FIG. 7. Thus, if the primary evaluation results of the common filter unit 53 are directly adopted, users such as specialists would find it difficult to properly discover true driver mutations, even by focusing particularly on the mutation statuses (sequence variations) of the rank MYC1, and would eventually have to check the mutation statuses (sequence variations) of the rank MYC2 equally with those of the rank MYC1. Therefore, to ensure that true drivers for the specific type of carcinoma or genetic disease that the user should focus on are accumulated in rank MYC1, the present embodiment adopts a seed gene filter that reevaluates (reclassifies) at least one mutation status (sequence variation) which has been classified into the rank MYC1 or MYC2 by the common filter unit 53.

The functional block incorporating the seed gene filter is the seed gene filter unit 54. Namely, the seed gene filter unit 54 performs reevaluation by reclassifying at least one mutation status (sequence variation), which has been classified into the rank MYC1 or MYC2 through the primary evaluation by the common filter unit 53, into the rank MYC1 or MYC2 using classification criteria set by the user, based on the type that the user should focus on, from among the plurality of types of carcinomas or genetic diseases. Detailed examples of the seed gene filter unit 54 will be described later with reference to FIGS. 6 to 8.

Meanwhile, true driver mutations may still be included in at least one mutation status (sequence variation) classified into the rank MYC3 through the primary evaluation by the common filter unit 53, and in at least one mutation status (sequence variation) reclassified into the rank MYC2 by the seed gene filter unit 54 (including those retained at the rank MYC2). Therefore, the present embodiment adopts a rescue filter for preventing users such as specialists from overlooking such true driver mutations.

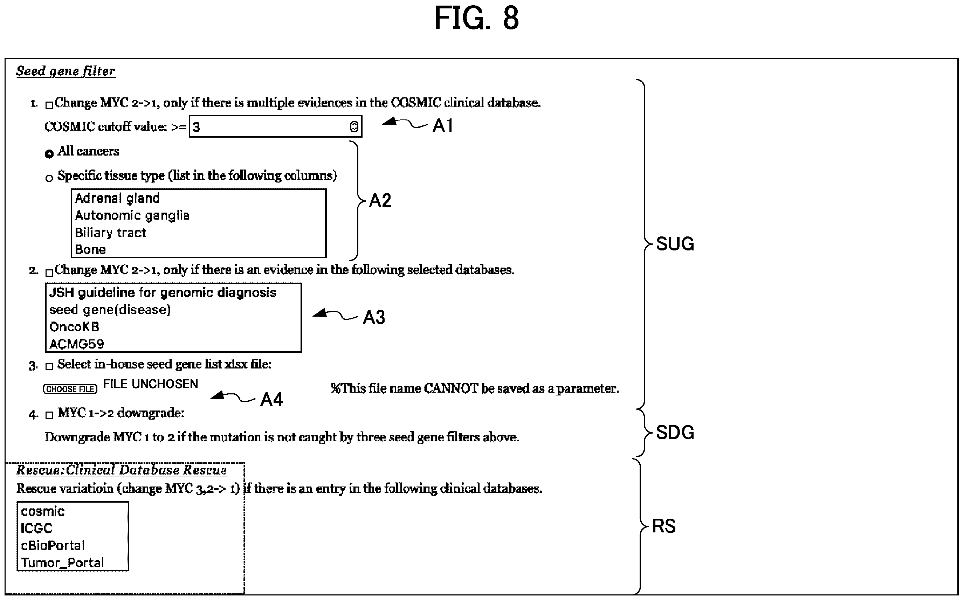

The functional block incorporating the rescue filter is the rescue filter unit 55. Namely, the rescue filter unit 55 performs reevaluation by either retaining in the rank MYC3 or MYC2, or reclassifying into the rank MYC1, at least one mutation status (sequence variation) which has been initially classified into the rank MYC3 by the common filter unit 53, and at least one mutation status (sequence variation) which has been reclassified into the rank MYC2 by the seed gene filter unit 54 (including those retained at the rank MYC2). Here, the classification method of the rescue filter unit 55 is not particularly limited, and may use rule-based methods with classification criteria different from those adopted by the common filter unit 53 and the seed gene filter unit 54, or may use models (such as AI models) obtained through machine learning. Detailed examples of the rescue filter unit 55 will be described later with reference to FIG. 8, etc.

The rank determination unit 56 determines a rank value representing the degree of likelihood of pathogenicity for each mutation status (sequence variation), based on the rank (one of the ranks MYC1 to MYC4) for each of the plurality of mutation statuses (sequence variations) outputted by the common filter unit 53, the seed gene filter unit 54, or the rescue filter unit 55. The rank determination unit 56 generates information associating each of the plurality of mutation statuses (sequence variations) with each rank value (hereinafter referred to as "analysis result information"), and provides the information to the analysis result output unit 57. The rank values representing the degree of likelihood of pathogenicity may be newly calculated values based on the ranks MYC1 to MYC4, but here the ranks MYC1 to MYC4 are directly adopted, for convenience of description.

The analysis result output unit 57 outputs the analysis result information via the output unit 17 (e.g., a display) of FIG. 1, or transmits the analysis result information to other devices (not illustrated) via the communication unit 19.

FIG. 4 illustrates an example of the configuration of analysis result information outputted by the analyzer illustrated in FIG. 2. As illustrated in FIG. 4, the analysis result information at least associates each mutation status (sequence variation) (each row in FIG. 4) with the number of the chromosome (Chr) in which the base sequence of the mutation status (sequence variation) has been identified, the start location (Start), the end location (End), the reference base sequence (Ref), the sequence variation (Alt), and the rank value (MYC).

In the example of the analysis result information illustrated in FIG. 4, the record information R related to the judgment is also associated with each mutation status (sequence variation) (each row in FIG. 4). The record information R indicates what classifications were made for the filters of the common filter unit 53, the seed gene filter unit 54, and the rescue filter unit 55 used in the analysis of the target mutation status (sequence variation) (e.g., parameter settings for each filter, or details of determination based on classification criteria). For example, when the mutation status is evaluated as pathogenic (classified into the rank MYC1), the users such as specialists referencing not only the rank values (MYC) but also the record information R can distinguish whether the reason for determination was mainly based on the time-series filter or the database filter. The users such as specialists can recognize whether the mutation status was initially classified into the rank MYC1 by the common filter unit 53 or reclassified into the rank MYC1 by the seed gene filter unit 54 or the rescue filter unit 55. This helps the users such as specialists better understand the nature of the mutation.

The example of the functional configuration of the analyzer 1 of FIG. 1 has been described above with reference to FIG. 2. Below, with reference to FIGS. 5 to 8, the detailed functional configuration of the common filter unit 53, the seed gene filter unit 54, and the rescue filter unit 55 in the analyzer 1 of FIG. 2 will be described sequentially.

FIG. 5 is a block diagram illustrating an example of the detailed functional configuration of the common filter unit in the analyzer of FIG. 2. In FIG. 5, the common filter unit 53 includes a basic filter 531, a time-series filter 532, a database filter 533, a functional prediction filter 534, and a quality filter 535.

Here, when the mutation status (sequence variation) as an analysis target can be determined to be benign, the basic filter 531 sets a rank (e.g., the rank MYC4) indicating a benign mutation. When the mutation status (sequence variation) as an analysis target cannot be determined to be benign, the basic filter 531 sets a rank (e.g., the rank MYC3) indicating that the mutation is not benign.

The cases where a mutation can be determined to be benign include: a relatively short duplication between the base sequence of a known mutation causing carcinogenesis and the mutant base sequence corresponding to the mutation status (sequence variation); an intron region including a mutation represented by the mutation status (sequence variation); a mutation status (sequence variation) registered in a database such as SNP databases accumulating normal mutations; or a mutation status (sequence variation) that can be determined to be benign based on the Gene Damage Index (GDI).

Here, the GDI is an indicator of the extent of damage accumulated in healthy individuals for each gene, suggesting the possibility that the gene, despite having accumulated significant damage (high diversity) in different individuals, is not considered pathogenic due to mutations.

From the setting receiving unit 52, the basic filter 531 receives settings including at least any one of: the threshold value for the length of the duplication between the base sequence of a known mutation causing carcinogenesis and the mutant base sequence corresponding to the mutation status (sequence variation); information specifying a database for determining whether the mutation is an SNP; or parameters for each database (e.g., the benign determination threshold value serving as a criterion for determining benignity, or comparison with the value registered in a database as the probability of being an SNP). The basic filter 531 determines whether the mutation status (sequence variation) as the analysis target is benign, based on the received settings.

Specifically, for example, the basic filter 531 sets a rank indicating a benign mutation when the sequence variation is located in a site referred to as a segmental duplication (hereinafter appropriately referred to as "segmental duplication region"). Segmental duplication refers to regions where genes have duplicated in adjacent sites during vertebrate evolution in a coherent region of 10 kb to 300 kb on the chromosome or have duplicated on completely separate and different genomes. When the sequence variation is located in a segmental duplication region, it is known that the sequence variation is likely to be a result of detection error when mapping the sequencing results to the reference, indicating a high likelihood of a false positive. Therefore, as mentioned above, the basic filter 531 sets a rank indicating a benign mutation when the sequence variation is located in a site referred to as segmental duplication. More specifically, when the sequence variation is located in a segmental duplication region, and the indicator indicating the degree of homology of the segmental duplication regions exceeds a threshold value, there is a high likelihood of a detection error, thus the basic filter 531 sets a rank indicating a benign mutation. For example, when the mutation represented by the mutation status (sequence variation) is located in an intron region, the basic filter 531 sets a rank indicating a benign mutation.

Furthermore, even if the above two criteria are not satisfied, the basic filter 531 may set a rank indicating a benign mutation, based on the results of searching a specified SNP database. For example, when the search results indicate that the mutation status (sequence variation) is registered in an SNP database, and the value registered as the probability of being an SNP exceeds the predetermined benign determination threshold value in the SNP database, the basic filter 531 sets a rank indicating a benign mutation.

Even if the previous criteria are not satisfied, the basic filter 531 may set a rank indicating a benign mutation by referring to the GDI of the gene where the mutation status (sequence variation) is located, and determining that the GDI exceeds a predetermined GDI threshold value.

Thus, the analyzer 1 can preliminarily screen out genes that cannot be cancer driver mutations (or sufficiently unlikely to be cancer driver mutations).

The basic filter 531 may receive settings from the setting receiving unit 52 regarding which predetermined criteria for judging benignity to utilize (or whether to pass all the mutation statuses (sequence variations) by setting the rank MYC3 without operating as the basic filter 531).

In this example, the basic filter 531 only determines whether the criteria set for use are satisfied.

When the basic filter 531 has passed the processing (setting the rank to MYC3), the time-series filter 532 refers to the information of mutation statuses (sequence variations) included in the time-series information corresponding to the mutation status (sequence variation) as the analysis target, and determines whether the same mutation exists in the time-series information extracted at different timings.

The time-series filter 532 uses the mutation status (sequence variation) as an analysis target and the corresponding mutation status (sequence variation) included in the time-series information. If the same mutation is present, the time-series filter 532 sets a rank indicating that there is a mutation to be addressed (for example, subtracting the first predetermined amount "1" from the current rank), and passes the processing to the quality filter 535. The first predetermined amount is, for example, the minimum value subtracted from or added to the rank of the mutation status (sequence variation) in a single calculation. In this example, since the basic filter 531 has passed the processing, the initial rank is MYC3, and when the time-series filter 532 determines that there is a mutation to be addressed, the rank is set to MYC2 by subtracting the first predetermined amount "1" from MYC3.

On the other hand, the time-series filter 532 uses the mutation status (sequence variation) as an analysis target and the corresponding mutation status (sequence variation) included in the time-series information. If the same mutation is not present, the time-series filter 532 retains the rank (retaining the initial rank MYC3) and passes the processing to the database filter 533.

The time-series filter 532 may receive settings from the setting receiving unit 52 regarding threshold values for depth, other sequence quality, mutation allele frequency, etc. For example, if the depth of the corresponding mutation status (sequence variation) included in the time-series information does not exceed the set threshold value (e.g., "20"), the time-series filter 532 retains the rank (retaining the initial rank MYC3) and passes the processing to the database filter 533 without determining whether the same mutation status (sequence variation) exists.

Further, in the present embodiment, if the data receiving unit 51 has not received time-series information (i.e., the case where the mutant base sequence information has been received only as an analysis target), the time-series filter 532 retains the rank (retaining the initial rank MYC3) and passes the processing to the database filter 533 without determining whether the same mutation status (sequence variation) exists.

If the setting receiving unit 52 has input the settings not to use the time-series filter 532, the time-series filter 532 retains the rank (retaining the initial rank MYC3) and passes the processing to the database filter 533 without determining whether the same mutation status (sequence variation) exists.

The database filter 533 determines whether the mutation status (sequence variation) as the analysis target is registered in a database that accumulates information on predetermined mutations to be addressed (e.g., COSMIC Cancer Database) by transmitting the information of the mutation status (sequence variation) to the database server. If the mutation status (sequence variation) is registered, the database filter 533 sets a rank indicating that there is a mutation to be addressed (for example, subtracting the first predetermined amount "1" from the current the rank) and passes the processing to the quality filter 535. In this example, the basic filter 531 passes the processing on the mutation status (sequence variation) as the analysis target, the time-series filter 532 further retains the rank and passes the processing, and this is the time when the database filter 533 makes determination. Thus, the database filter 533 sets the rank to MYC2 by subtracting the first predetermined amount "1" from MYC3, and passes the processing to the quality filter 535.

If the mutation status (sequence variation) as an analysis target is not registered in the database that accumulates information on mutations to be addressed, the database filter 533 retains the rank and passes the processing to the functional prediction filter 534. In this example, the rank is retained at MYC3.

The database filter 533 receives settings from the setting receiving unit 52 regarding which databases to use as the database that accumulates information on mutations to be addressed.

These settings may indicate using a plurality of databases, and if the mutation status (sequence variation) as an analysis target is registered in any one of the databases that accumulate information on mutations to be addressed, the database filter 533 sets a rank indicating that there is a mutation to be addressed.

The functional prediction filter 534 refers to a database that provides evaluation on the pathogenicity of mutations, and if the mutation status (sequence variation) as an analysis target is registered as pathogenic in the database, the functional prediction filter 534 sets a rank indicating that there is a pathogenic mutation (for example, subtracting the first predetermined amount "1" from the current rank), and passes the processing to the quality filter 535.

Widely known databases, such as SIFT and PolyPhen2, evaluate the pathogenicity of mutations. Some of these databases provide multistage evaluation on the presence or absence of pathogenicity, and in such cases, for example, at the stage where pathogenicity is suspected, the functional prediction filter 534 sets a rank indicating that there is a pathogenic mutation (for example, subtracting the first predetermined amount "1" from the current rank), and passes the processing to the quality filter 535.

In this example, the basic filter 531 passes the processing on the mutation status (sequence variation) as the analysis target, the time-series filter 532 retains the rank and passes the processing, the database filter 533 retains the rank and passes the processing, and this is the time when the functional prediction filter 534 makes determination. Thus, the functional prediction filter 534 sets the rank to MYC2 by subtracting the first predetermined amount "1" from MYC3, and passes the processing to the quality filter 535.

The functional prediction filter 534 refers to a database that provides evaluation on the pathogenicity of mutations, and if the mutation status (sequence variation) as an analysis target is not registered as pathogenic in the database that provides evaluation on the pathogenicity of mutations (or is registered as unknown, benign, or presumed benign), the functional prediction filter 534 retains the rank and passes the processing to the quality filter 535. In this example, the rank is retained at MYC3.

In this case as well, the functional prediction filter 534 receives settings from the setting receiving unit 52 regarding which databases to use.

The quality filter 535 evaluates the quality of the sequencing process of the mutation status (sequence variation) as an analysis target, such as depth and other indicators of sequencing quality. Widely known quality indicators include depth and the count number of the mutation status (sequence variation). The quality filter 535 combines these indicators (or receives the combination from the setting receiving unit 52 and follows the received combination of indicators) to evaluate the quality. In the case of combining a plurality of indicators, if all indicators satisfy the high-quality criteria, the quality filter 535 determines that the quality is sufficient.

If the quality filter 535 determines that the sequencing quality of the mutation status (sequence variation) as the analysis target is sufficient (sufficiently high), the quality filter 535 sets a rank indicating that the determination is appropriate (for example, subtracting the first predetermined amount "1" from the current rank), and outputs the rank to the seed gene filter unit 54, the rescue filter unit 55, and the rank determination unit 56. If the quality filter 535 cannot determine that the sequencing quality of the mutation status (sequence variation) as the analysis target is sufficient (sufficiently high), the quality filter 535 retains the rank and outputs the rank to the seed gene filter unit 54, the rescue filter unit 55, and the rank determination unit 56.

The detailed functional configuration of the common filter unit 53 in the analyzer 1 of FIG. 2 has been described with reference to FIG. 5. Next, the detailed functional configuration of the seed gene filter unit 54 in the analyzer 1 of FIG. 2 will be described with reference to FIGS. 6 to 8.

FIG. 6 is a block diagram illustrating an example of the detailed functional configuration of the seed gene filter unit in the analyzer of FIG. 2. In FIG. 6, the seed gene filter unit 54 includes a seed gene filter 541, a parameter setting receiving unit 542, and a seed gene information acquisition unit 543.

The seed gene filter 541 is a filter that reclassifies each of at least one mutation status (sequence variation), which has been classified into the rank MYC1 or MYC2 through the primary evaluation by the common filter unit 53, into the rank MYC1 or MYC2 using predetermined classification criteria. Here, reclassification into the rank MYC1 (including retaining the rank MYC1) is referred to as "upgrade". Conversely, reclassification into the rank MYC2 (including retaining the rank MYC1) is referred to as "downgrade". Specifically, for example, if the mutation status (sequence variation) as a classification target is classified into the rank MYC2, the seed gene filter 541 upgrades the rank to MYC1 when the classification target satisfies the classification criteria, and downgrades (retains) the rank to (at) the rank MYC2 when the classification target does not satisfy the classification criteria. Similarly, for example, if the mutation status (sequence variation) as a classification target is classified into the rank MYC1, the seed gene filter 541 upgrades (retains) the rank to (at) the rank MYC1 when the classification target satisfies the classification criteria, and downgrades the rank to MYC2 when the classification target does not satisfy the classification criteria. In this example, in order to facilitate understanding, the same type of classification criterion is used for both the classification target classified into the rank MYC1 and the classification target classified into the rank MYC2, but this is not limiting. For example, the type-1 classification criteria may be used for the target classification classified into the rank MYC1, while the type-2 classification criteria may be used for the classification target classified into the rank MYC2. As described later with reference to FIG. 8, the seed gene filter 541 can be set by users such as specialists to function as a filter performing only upgrades or downgrades.

The parameter setting receiving unit 542 receives parameters for setting the classification criteria of the seed gene filter 541. For example, the parameter setting receiving unit 542 receives parameters specified by the user, based on the type that the user should focus on, from among the plurality of types of carcinomas or genetic diseases. The parameter setting receiving unit 542 sets the classification criteria of the seed gene filter 541, based on the received parameters. For example, the parameter setting receiving unit 542 may receive parameters indicating the "database or list" appropriate for the type that the user should focus on, from among the plurality of types of carcinomas or genetic diseases. In such cases, for example, the classification criteria of the seed gene filter 541 are set by the parameter setting receiving unit 542, based on the parameter indicating being registered in the "database or list". Additionally, for example, the parameter setting receiving unit 542 may receive parameters indicating the type that the user should focus on, from among the plurality of types of carcinomas or genetic diseases. In such cases, the classification criteria of the seed gene filter 541 are set by the parameter setting receiving unit 542, based on the parameter indicating that the type of carcinoma or genetic disease is registered in the "database or list". Furthermore, for example, the parameter setting receiving unit 542 may receive parameters indicating the minimum number of registrations in the "database or list". In such cases, the classification criteria of the seed gene filter 541 are set by the parameter setting receiving unit 542, based on the parameter indicating that the number of registrations in the "database or list" is at least the minimum number of registrations specified by the parameter. Detailed examples of parameter settings will be described later with reference to FIG. 8.

The seed gene information acquisition unit 543 adopts, as seed gene information, the information used by the seed gene filter 541 for determining whether the mutation status (sequence variation) as a classification target satisfies the classification criteria. The seed gene information can include the "database or list" itself or the results of searching the "database or list". For example, regarding the mutation which has been reported (sampled) for a particular type of carcinoma or genetic disease, the database includes coordinates (locations) on the reference genome, statistical information on the mutation, and information on the case. Specifically, for example, regarding the reported mutation, the database includes the statistical information on how many reports (samples) describe that "a particular base of a predetermined gene at a predetermined coordinate has mutated to another base (any base)". Additionally, the list includes information on mutations reported (sampled) for a specific type of carcinoma or genetic disease for each sample. Thus, the database or list includes information on the reports (samples) on predetermined types of carcinoma or genetic diseases, such as "a particular base of a predetermined gene at a predetermined coordinate has mutated to another base (any base)" or "a base of a sequence (expression regulatory sequence) that determines when and where a gene works has mutated to another base (any base)". Expression regulatory sequences include, for example, enhancers, promoters, non-protein-coding RNAs, etc. In other words, the information on base mutations in the specified coordinates of the gene (base sequence) or expression regulatory sequences included in the seed gene information is compared with the sequence variation for consideration. Thus, the seed gene filter 541 uses the seed gene information to determine whether the mutation status (sequence variation) as a classification target satisfies the classification criteria. The seed gene filter 541 upgrades the rank if the mutation status satisfies the classification criteria, and downgrades the rank if the mutation status does not satisfy the classification criteria.

The adoption of such a seed gene filter unit 54 offers the following first to third advantages:
First, for drug approval applications in Japan, it is only necessary to obtain approval for the settings of the parameters that may be received by the parameter setting receiving unit 542, regardless of the type of carcinoma or genetic disease. Second, the seed gene information can be easily updated. Third, users such as specialists can easily perform reanalysis (using the seed gene filter 541) based on the settings (of parameters).

Further, the technical significance of adopting such a seed gene filter unit 54 will be described with reference to FIG. 7. FIG. 7 is a schematic diagram illustrating the significance of adopting the seed gene filter unit in the analyzer illustrated in FIG. 2. The bar graph on the left side of FIG. 7 illustrates the number of sequence variations of the ranks MYC1 and MYC2 as a result of the primary evaluation by the common filter unit 53. If the primary evaluation results of the common filter unit 53 are directly adopted, there will be a problem of having a large number of sequence variations of the rank MYC1 (making interpretation by users such as specialists inefficient). In other words, as described above, the primary evaluation by the common filter unit 53 may result in a high number of true driver mutations in the sequence variations of the rank MYC2, or conversely, a high number of false positives in the sequence variations of the rank MYC1, depending on the type of carcinoma or genetic disease. Thus, if the primary evaluation results of the common filter unit 53 are directly adopted, users such as specialists would find it difficult to properly discover true driver mutations, even by focusing particularly on the mutation statuses (sequence variations) of the rank MYC1, and would eventually have to check the mutation statuses (sequence variations) of the rank MYC2 equally with those of the rank MYC1, which is the problem. The cause of this problem is that the common filter unit 53 is configured with filters using common classification criteria for all cancers and genetic diseases, and does not weight the important genes or expression regulatory sequences for the types of carcinomas or genetic diseases that the users such as specialists focus on.

The seed gene filter unit 54 is adopted to solve this problem. The bar graph on the right side of FIG. 7 illustrates the results of reevaluation by the seed gene filter unit 54 after the primary evaluation by the common filter unit 53. As described above, the seed gene filter unit 54 sets classification criteria using the parameters specified by the users, based on the type of carcinoma or genetic disease that the users such as specialists focus on, and obtains seed gene information. The seed gene filter 541 sequentially sets each of the mutation statuses (sequence variations) of the ranks MYC1 and MYC2 illustrated on the left side of FIG. 7 as a classification target, uses the seed gene information to determine whether the classification target satisfies the classification criteria, upgrades the rank if the classification target satisfies the classification criteria, and downgrades the rank if the classification target does not satisfy the classification criteria. As a result, as illustrated in the bar graph on the right side of FIG. 7, true driver mutations for the types of carcinomas or genetic diseases that the users such as specialists focus on are accumulated in the rank MYC1. Thus, the users such as specialists can focus on verifying the rank MYC1, allowing for reducing the likelihood of missing true driver mutations.

FIG. 8 illustrates an example of the screen for parameter input to the seed gene filter unit and the rescue filter unit in the analyzer of FIG. 2.

In the screen example in FIG. 8, a region SUG is for the users such as specialists to specify parameters for setting the classification criteria for upgrading by the seed gene filter 541. In the region SUG, the users such as specialists can specify parameters from three perspectives for setting the classification criteria for upgrading by the seed gene filter 541.

The first perspective for setting the classification criteria, indicated by "1" in FIG. 8, involves using COSMIC as the database. When setting the classification criteria from the first perspective, the users such as specialists check the right box labeled "1" in FIG. 8. Two parameters can be specified from the first perspective. The first parameter is specified in the field A1. The field A1 is a field in which the users such as specialists specify (input) the cutoff value (minimum number of registrations) for COSMIC. The parameter setting receiving unit 542 sets a classification criterion that the number of registrations in COSMIC is at least the cutoff value (minimum number of registrations), as the classification criterion for upgrading by the seed gene filter 541. The second parameter is specified in the field A2. The field A2 is a field in which the users such as specialists specify (input) whether the classification criteria should apply to all carcinomas or be limited to the carcinomas specified by the users such as specialists and registered in COSMIC. When applying the classification criteria to all carcinomas, the users such as specialists check the left box labeled "All cancers". In this case, the parameter setting receiving unit 542 sets a classification criterion that the number of registrations for all carcinomas in COSMIC is at least the cutoff value (minimum number of registrations), as the classification criterion for upgrading by the seed gene filter 541. Conversely, when specifying a particular carcinoma, the users such as specialists check the left box labeled "Specific tissue type", and further specify at least one from the plurality of types of carcinomas in the list below. In this case, the parameter setting receiving unit 542 sets a classification criterion that the number of registrations in COSMIC for at least one type of carcinoma specified by the users such as specialists is at least the cutoff value (minimum number of registrations), as the classification criterion for upgrading by the seed gene filter 541.

The second perspective for setting the classification criteria, indicated by "2" in FIG. 8, involves using a database or list other than COSMIC. When setting the classification criteria from the second perspective, the users such as specialists check the right box labeled "2" in FIG. 8. In the second perspective, the users such as specialists specify databases of genes (such as genes listed in guidelines) or cancers to be weighted in the field A3, as parameters. In this case, the parameter setting receiving unit 542 sets a classification criterion of being registered in the "databases of genes (such as genes listed in guidelines) or cancers to be weighted" specified by the users such as specialists, as the classification criterion for upgrading by the seed gene filter 541.

The third perspective for setting the classification criteria, indicated by "3" in FIG. 8, involves using genes specified by the users such as specialists themselves. When setting the classification criteria from the third perspective, the users such as specialists check the right box labeled "3" in FIG. 8. In the third perspective, the users such as specialists specify the genes to be weighted in the field A4. In this case, the parameter setting receiving unit 542 sets a classification criterion corresponding to the genes or expression regulatory sequences to be weighted as specified by the users such as specialists themselves, as the classification criterion for upgrading by the seed gene filter 541.

An example of the classification criteria for upgrading by the seed gene filter 541 has been described from the three perspectives. The classification criteria from the three perspectives are not mutually exclusive, and at least two classification criteria can be specified in combination. When at least two classification criteria are specified (i.e., when at least two of the boxes labeled "1" to "3" are checked), an OR condition is adopted, which means that the condition is considered satisfied if any one of the at least two classification criteria is satisfied. Specifically, if the mutation status (sequence variation) as a classification target is classified into the rank MYC2, the seed gene filter 541 upgrades the rank to MYC1 if the classification target satisfies any one of the at least two classification criteria. Similarly, if the mutation status (sequence variation) as a classification target is classified into the rank MYC1, the seed gene filter 541 upgrades (retains) the rank to (at) MYC1 if the classification target satisfies any one of the at least two classification criteria.

In the screen example in FIG. 8, a region SDG is for the users such as specialists to specify parameters for activating the downgrade function of the seed gene filter 541. In order to activate the downgrade function of the seed gene filter 541, the users such as specialists check the right box labeled "4" in FIG. 8. In this case, when at least one of the classification criteria of the three perspectives is specified (i.e., at least one of the boxes labeled "1" to "3" is checked), and none of the classification criteria are satisfied, the rank will be downgraded. Specifically, if the mutation status (sequence variation) as the classification target is classified into the rank MYC2, the seed gene filter 541 downgrades (retains) the rank to (at) MYC2 if none of the classification criteria are satisfied. Similarly, if the mutation status (sequence variation) as the classification target is classified into the rank MYC1, the seed gene filter 541 downgrades the rank to MYC2 if none of the classification criteria are satisfied.

In the screen example in FIG. 8, a region RS is for the users such as specialists to specify parameters for setting the classification criteria for the rescue filter unit 55. The users such as specialists specify at least one database or list as a parameter. In this case, the rescue filter unit 55 sets a classification criterion of being registered in the at least one database or list specified by the users such as specialists, as the classification criterion for the rescue filter unit 55. The rescue filter unit 55 sequentially sets at least one mutation status (sequence variation) classified into the rank MYC3 through the primary evaluation by the common filter unit 53, and at least one mutation status (sequence variation) reclassified into the rank MYC2 by the seed gene filter unit 54 (including those retained at the rank MYC2), as classification targets. The rescue filter unit 55 reclassifies the rank to MYC1 if the mutation status (sequence variation) as classification targets satisfies the classification criteria, and retains the rank MYC3 or MYC2 if the mutation status does not satisfy the classification criteria.

In the screen example in FIG. 8, the rescue filter unit 55 adopts a rule-based method. However, the classification method by the rescue filter unit 55 is not limited to this, and may use a model (such as an AI model) obtained through machine learning as described above. The rescue filter unit 55 in the case of adopting this method is described below.

A learning device (not illustrated) executes predetermined machine learning, using a plurality of learning information sets, on predetermined nucleic acids. These learning information sets include information indicating known sequence variations that pose a risk of harm, and clinical significance information on at least some variations from public databases, human genetic polymorphism databases, drug-gene interaction and druggable genome resource databases, and drug response databases. This allows the learning device to generate or update a model (such as an AI model) that reclassifies and outputs a predetermined input sequence variation of the rank MYC2 or MYC3 into MYC1, or outputs the sequence variation retained at the rank MYC2 or MYC3. Here, updating means relearning by adding learning information set. The learning device may be provided as part of the analyzer 1 or as a device separate from the analyzer 1.

For example, public databases such as ClinVar (database of human genome variations and associated diseases, including genetic diseases) and the aforementioned COSMIC can be adopted. For example, dbsnp can be adopted as a human gene polymorphism database. For example, DGId can be adopted as a drug-gene interaction and druggable genome resource database. For example, PharmGKB or OncoKB can be adopted as a drug response database.

In this case, the rescue filter unit 55 sequentially sets at least one mutation status (sequence variation) classified into the rank MYC3 through the primary evaluation by the common filter unit 53, and at least one mutation status (sequence variation) reclassified into the rank MYC2 by the seed gene filter unit 54 (including those retained at the rank MYC2), as classification targets. The rescue filter unit 55 inputs the mutation status (sequence variation) as a classification target into the model (such as an AI model) generated or updated by the learning device, reclassifies the mutation status (sequence variation) into the rank MYC1 if the output from the model is the rank MYC1, and retains the rank MYC3 or MYC2 in other cases.

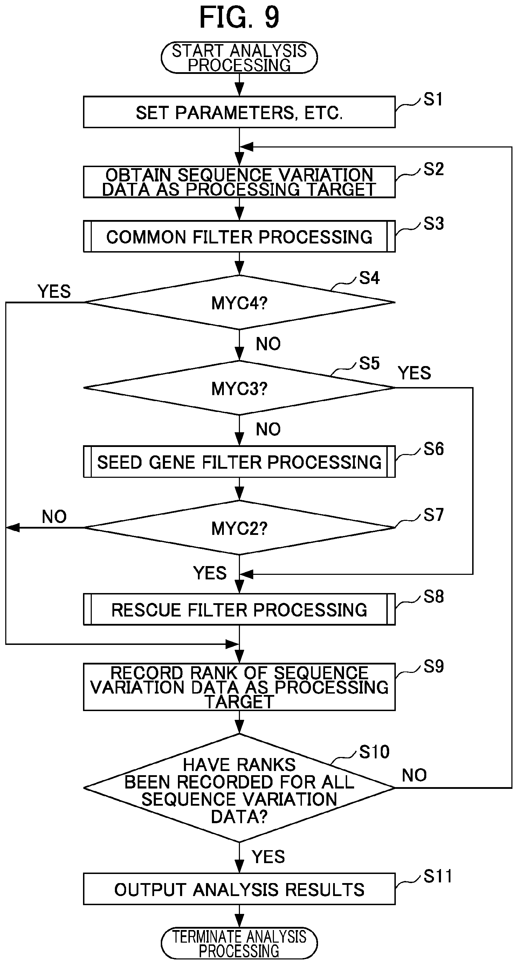

The functional configuration of the analyzer 1 has been described with reference to FIGS. 6 to 8. Next, the processing of the analyzer 1 will be described with reference to FIGS. 9 and beyond.

FIG. 9 is a flowchart illustrating an example of the analysis processing flow in the analyzer with the functional configuration of FIG. 6.

In Step S1, the setting receiving unit 52 and the parameter setting receiving unit 542 receive the settings of parameters, etc.

In Step S2, the data receiving unit 51 determines a predetermined mutation status (sequence variation data) as a processing target, from among the mutant base sequence information extracted through sequence alignment from the genetic information of the sample as an analysis target.

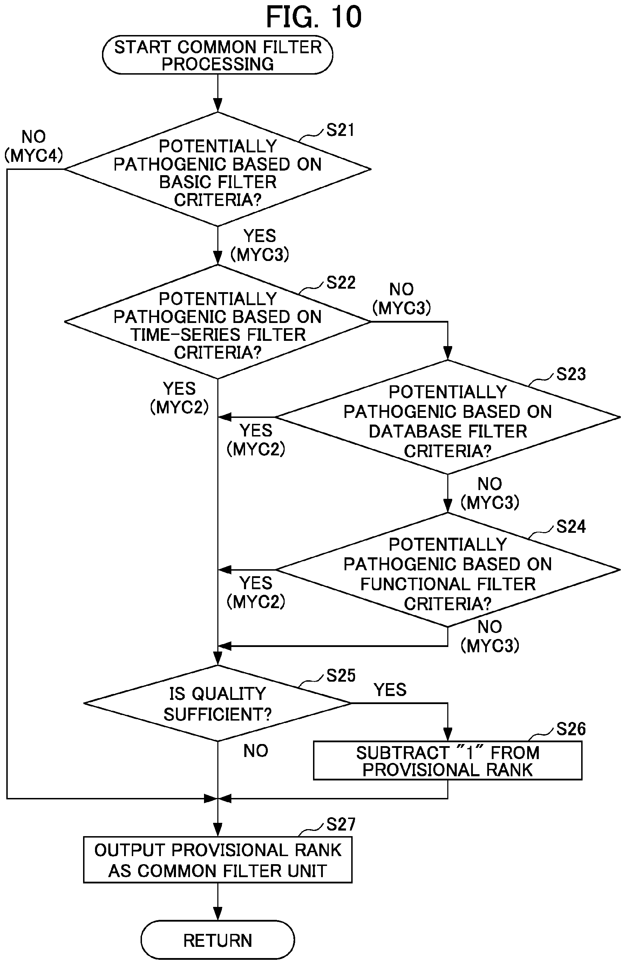

In Step S3, the common filter unit 53 executes common filter processing on the sequence variation data as the processing target and outputs a provisional rank of the processing target. Details of the common filter processing will be described with reference to FIG. 10.

In Step S4, the analyzer 1 determines whether the provisional rank of the sequence variation data as the processing target (output from the common filter unit 53) is rank MYC4.

If the provisional rank (output from the common filter unit 53) is MYC4, the determination in Step S4 is "YES", and the processing proceeds to Step S9. In Step S9, the rank determination unit 56 records the provisional rank MYC4 for the sequence variation data as the processing target. Subsequently, the processing proceeds to Step S10. The processing from Step S10 onward will be described later.

Meanwhile, if the provisional rank (output from the common filter unit 53) is any one of MYC1 to MYC3, the determination in Step S4 is "NO", and the processing proceeds to Step S5. In Step S5, the analyzer 1 determines whether the provisional rank of the sequence variation data as the processing target (output from the common filter unit 53) is rank MYC3.

If the provisional rank (output from the common filter unit 53) is MYC3, the determination in Step S5 is "YES", and the processing proceeds to Step S8. The processing in Step S8 will be described later.

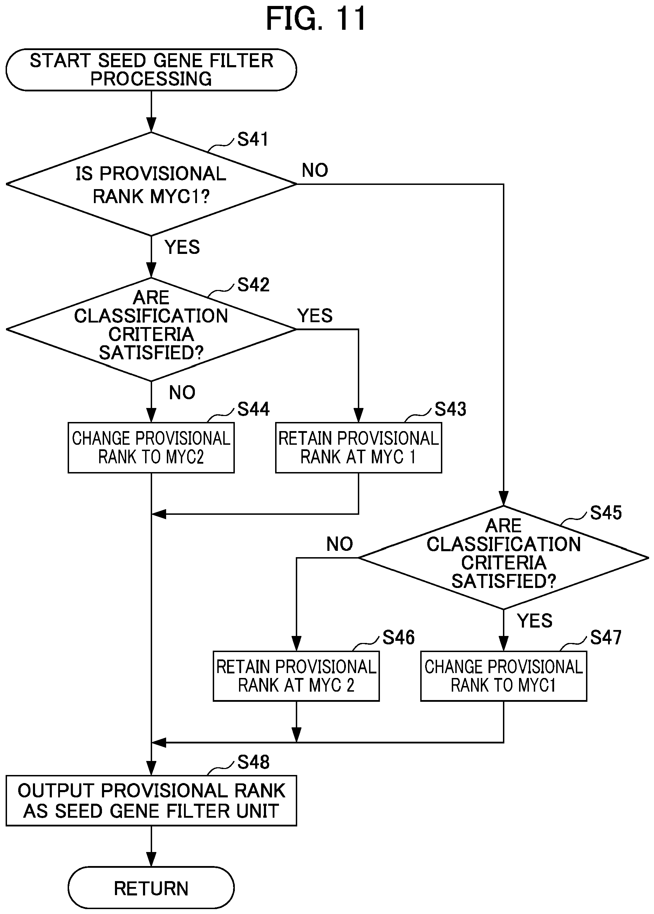

Meanwhile, if the provisional rank (output from the common filter unit 53) is either MYC1 or MYC2, the determination in Step S5 is "NO", and the processing proceeds to Step S6. In Step S6, the seed gene filter unit 54 executes seed gene filter processing on the sequence variation data as the processing target. Details of the seed gene filter processing will be described with reference to FIG. 11.

In Step S7, the analyzer 1 determines whether the provisional rank of the sequence variation data as the processing target (output from the seed gene filter unit 54) is rank MYC2.

If the provisional rank (output from the seed gene filter unit 54) is MYC1, the determination in Step S7 is "NO", and the processing proceeds to Step S9. In Step S9, the rank determination unit 56 records the provisional rank MYC1 for the sequence variation data as the processing target. Subsequently, the processing proceeds to Step S10. The processing from Step S10 onward will be described later.

Meanwhile, if the provisional rank (output from the seed gene filter unit 54) is MYC2, the determination in Step S7 is "YES", and the processing proceeds to Step S8.

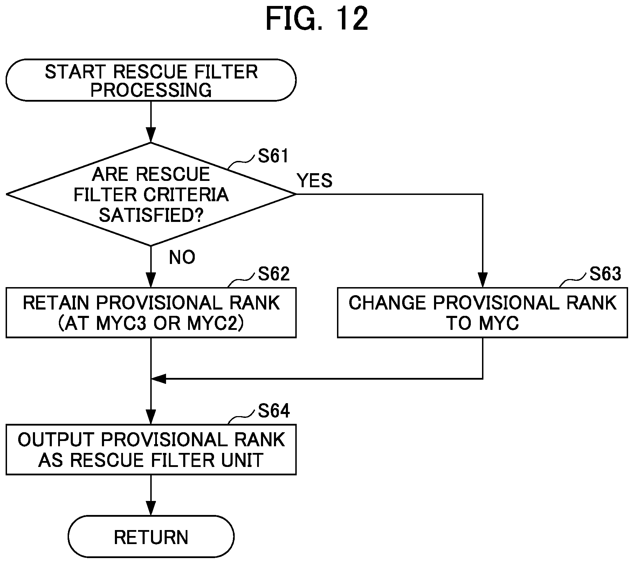

In this manner, if the provisional rank outputted from the seed gene filter unit 54 is MYC2 ("YES" in Step S7) or the provisional rank outputted from the common filter unit 53 is MYC3 ("YES" in Step S5), the rescue filter unit 55 executes rescue filter processing on the sequence variation data as the processing target in Step S8. Details of the rescue filter processing will be described with reference to FIG. 12. In Step S9, the rank determination unit 56 records the provisional rank (MYC1, MYC2, or MYC3) of the sequence variation data as the processing target, which is outputted from the rescue filter unit 55.

Thus, when the provisional rank of the sequence variation data as the processing target is recorded in Step S9, the processing proceeds to Step S10.

In Step S10, the analyzer 1 determines whether the ranks have been recorded for all sequence variation data. If there are sequence variation data for which the rank has not been recorded, the determination in Step S10 is "NO", the processing returns to Step S2, and the subsequent processing is repeated. As a result of repeating the loop processing of Steps S2 to S10 "NO", once the ranks for all sequence variation data have been recorded, the determination in Step S10 is "YES", and the processing proceeds to Step S11.

In Step S11, the analysis result output unit 57 generates analysis result information, and outputs the information from the output unit 17 (such as a display) in FIG. 1 or outputs and transmits the information to another device (not illustrated) via the communication unit 19. Consequently, the analysis processing is terminated.

Next, regarding the analysis processing, details of the common filter processing in Step S3, the seed gene filter processing in Step S6, and the rescue filter processing in Step S8 will be described in sequence.

FIG. 10 is a flowchart illustrating the details of the common filter processing in Step S3, regarding the analysis processing in FIG. 9.

In Step S21, the basic filter 531 determines whether the sequence variation data as the processing target is potentially pathogenic based on the basic filter criteria. If the mutation status (sequence variation) as the processing target is determined not to be potentially pathogenic based on the basic filter criteria, the determination in Step S21 is "NO", the provisional rank is set to MYC4, and the processing proceeds to Step S27. In Step S27, the common filter unit 53 outputs the provisional rank as the common filter unit. Consequently, the common filter processing in Step S3 of FIG. 9 is terminated, and the processing proceeds to Step S4.

If the mutation status (sequence variation) as the processing target is determined to be potentially pathogenic based on the basic filter criteria, the determination in Step S21 is "YES", the provisional rank is set to MYC3, and the processing proceeds to Step S22.

In Step S22, the time-series filter 532 determines whether the sequence variation data as the processing target is potentially pathogenic based on the time-series filter criteria. If the mutation status (sequence variation) as the processing target is determined to be potentially pathogenic based on the time-series filter criteria, the determination in Step S22 is "YES", the provisional rank is set to MYC2, and the processing proceeds to Step S25. The processing from Step S25 onward will be described later. If the mutation status (sequence variation) as the processing target is determined to be potentially pathogenic based on the time-series filter criteria, the determination in Step S22 is "NO", the provisional rank is set to MYC3, and the processing proceeds to Step S23.

In Step S23, the database filter 533 determines whether the sequence variation data as the processing target is potentially pathogenic based on the database filter criteria. If the mutation status (sequence variation) as the processing target is determined to be potentially pathogenic based on the database filter criteria, the determination in Step S23 is "YES", the provisional rank is set to MYC2, and the processing proceeds to Step S25. The processing from Step S25 onward will be described later. If the mutation status (sequence variation) as the processing target is determined to be potentially pathogenic based on the time-series filter criteria, the determination in Step S23 is "NO", the provisional rank is set to MYC3, and the processing proceeds to Step S24.

In Step S24, the functional prediction filter 534 determines whether the sequence variation data as the processing target is potentially pathogenic based on the functional filter criteria. If the mutation status (sequence variation) as the processing target is determined to be potentially pathogenic based on the functional filter criteria, the determination in Step S24 is "YES", the provisional rank is set to MYC2, and the processing proceeds to Step S25. If the mutation status (sequence variation) as the processing target is determined to be potentially pathogenic based on the functional filter criteria, the determination in Step S24 is "NO", the provisional rank is set to MYC3, and the processing proceeds to Step S25.

In Step S25, the quality filter 535 determines whether the quality is sufficient. If the quality of the results of the processing in Steps S21 to S24 (the filter results of the basic filter 531, the time-series filter 532, the database filter 533, and the functional prediction filter 534) is sufficient, the determination in Step S25 is "YES", and the processing proceeds to Step S26. Since the quality filter 535 has determined that the quality is sufficient, the first predetermined amount "1" is subtracted from the provisional rank in Step S26.

If the quality of the results of the processing in Steps S21 to S24 (the filter results of the basic filter 531, the time-series filter 532, the database filter 533, and the functional prediction filter 534) is not sufficient, the determination in Step S25 is "NO", and the processing proceeds to Step S27.

In Step S27, the common filter unit 53 outputs the provisional rank as the common filter unit. Consequently, the common filter processing in Step S3 of FIG. 9 is terminated, and the processing proceeds to Step S4.

FIG. 11 is a flowchart illustrating the details of the seed gene filter processing in the analysis processing of FIG. 9. In Step S41, the seed gene filter unit 54 determines whether the provisional rank of the sequence variation data as the processing target is MYC1. If the provisional rank is MYC1, the determination in Step S41 is "YES", and the processing proceeds to Step S42. If the provisional rank is MYC2, the determination in Step S41 is "NO", and the processing proceeds to Step S45. The processing from Step S45 onward will be described later.

In Step S42, the seed gene filter 541 determines whether the sequence variation data as the processing target satisfies the classification criteria for upgrading. If the mutation status (sequence variation) as the processing target satisfies the classification criteria for upgrading, the determination in Step S42 is "YES", and the processing proceeds to Step S43. In Step S43, the seed gene filter 541 retains (upgrades) the provisional rank at MYC1. Subsequently, the processing proceeds to Step S48. The processing in Step S48 will be described later.

If the mutation status (sequence variation) as the processing target does not satisfy the classification criteria for upgrading, the determination in Step S42 is "NO", and the processing proceeds to Step S44. In Step S44, the seed gene filter 541 changes (downgrades) the provisional rank to MYC2. Subsequently, the processing proceeds to Step S48. The processing in Step S48 will be described later.

In Step S45, the seed gene filter 541 determines whether the sequence variation data as the processing target satisfies the classification criteria for upgrading.

If the mutation status (sequence variation) as the processing target satisfies the classification criteria for upgrading, the determination in Step S45 is "YES", and the processing proceeds to Step S46. In Step S46, the seed gene filter 541 retains (downgrades) the provisional rank at MYC2. Subsequently, the processing proceeds to Step S48. The processing in Step S48 will be described later.

If the mutation status (sequence variation) as the processing target does not satisfy the classification criteria for upgrading, the determination in Step S45 is "NO", and the processing proceeds to Step S47. In Step S47, the seed gene filter 541 changes (upgrades) the provisional rank to MYC1. Subsequently, the processing proceeds to Step S48.

In Step S48, the seed gene filter unit 54 outputs the provisional rank as the seed gene filter unit. Consequently, the common filter processing in Step S6 of FIG. 9 is terminated, and the processing proceeds to Step S7.

FIG. 12 is a flowchart illustrating the details of the rescue filter processing in Step S8 of the analysis processing in FIG. 9. In Step S61, the rescue filter unit 55 determines whether the sequence variation data as the processing target satisfies the rescue filter criteria. If the mutation status (sequence variation) as the processing target does not satisfy the rescue filter criteria, the determination in Step S61 is "NO", and the processing proceeds to Step S62. In Step S62, the rescue filter unit 55 retains (downgrades) the provisional rank to MYC3 or MYC2. Subsequently, the processing proceeds to Step S64. The processing in Step S64 will be described later.

If the mutation status (sequence variation) as the processing target satisfies the rescue filter criteria, the determination in Step S61 is "YES", and the processing proceeds to Step S63. In Step S63, the rescue filter unit 55 changes (upgrades) the provisional rank to MYC1. Subsequently, the processing proceeds to Step S64.

In Step S64, the rescue filter unit 55 outputs the provisional rank as the rescue filter unit. Consequently, the rescue filter processing in Step S8 of FIG. 9 is terminated, and the processing proceeds to Step S9.

The above rescue filter processing is an example of the processing of the rescue filter unit 55, which adopts a rule-based approach. That is, when adopting a classification method using a model (such as an AI model) obtained by machine learning, the rescue filter processing becomes a simple process of inputting the sequence data as the processing target to the model and outputting the rank from the model.

Although an embodiment of the present invention has been described above, the present invention is not limited to the aforementioned embodiment, and modifications, improvements, and the like that can achieve the object of the present invention are included in the present invention.

For example, the common filter unit 53 is not particularly limited to the example in FIG. 5 and can take various forms including the following configurations. Specifically, for example, the common filter unit 53 can also include the configuration illustrated in FIG. 13.

FIG. 13 is a block diagram illustrating another example of the detailed functional configuration of the common filter unit 53 in the analyzer in FIG. 2, different from the example in FIG. 5.

The common filter unit in the example in FIG. 13 is useful for analyzing the following mutation statuses (sequence variations). Therefore, the following is described based on analyzing the following mutation statuses (sequence variations). As a premise, it is known that the fusion of two specific genes due to chromosomal translocation or inversion can cause cancer cell proliferation. For example, the BCR-ABL fusion gene, which is the fusion of the BCR gene and the ABL gene due to chromosomal translocation, is known to proliferate leukemia cells.

The common filter unit 53 includes a basic filter 531, a time-series filter 532, a fusion gene filter 536, a conserved location filter 537, a structure filter 538, and a quality filter 539. The base sequences encoding a plurality of combinations of candidate genes known to cause driver mutations in fusion genes formed by specific combinations of two candidate genes are stored in a region of the storage unit 18 for each fusion gene. For example, the base sequences encoding the BCR gene and the ABL gene are stored in a region of the storage unit 18. That is, the analyzer 1 can obtain and use the following information for information processing.

The analyzer 1 obtains the base sequences of two candidate genes as candidate driver mutations in a fusion gene formed by a specific combination of the candidate genes (hereinafter referred to as first fusion genes) for each of the first fusion genes. In the example in FIG. 13 where the common filter unit 53 is adopted, the analyzer 1 obtains the base sequences of two candidate genes, which are included in the plurality of first fusion genes stored in the storage unit 18, from the storage unit 18 for each of the first fusion genes.

The external server (not illustrated) may store the base sequences encoding the candidate genes for the plurality of first fusion genes. The analyzer 1 may obtain the base sequences encoding the two candidate genes for the first fusion genes from the external server for each of the first fusion genes via the communication unit 19.

Fusion genes, formed by the fusion of a specific candidate gene with another gene, can sometimes cause cancer cell proliferation. For example, fusion genes formed by the fusion of the ALK gene and another gene are known to cause cancer cell proliferation. The storage unit 18 stores the base sequences of a plurality of candidate genes that are candidate driver mutations in fusion genes (hereinafter also referred to as second fusion genes) formed by the fusion with another gene.

The analyzer 1 obtains the base sequences of candidate genes that are candidate driver mutations in the second fusion genes formed by the fusion with another gene. For example, the analyzer 1 obtains the base sequences of candidate genes for the plurality of second fusion genes from the storage unit 18. The analyzer 1 may obtain the base sequences of candidate genes for the plurality of second fusion genes from an external server via the communication unit 19.

The analyzer 1 obtains conserved sequence location information, which indicates the locations of conserved sequences which are base sequences conserved between the genomes of different species. For example, the analyzer 1 obtains the conserved sequence location information from the storage unit 18. The analyzer 1 may obtain the conserved sequence location information from an external server via the communication unit 19.

The basic filter 531 is the same as the one in FIG. 2, except for not executing processing specific to single nucleotide polymorphisms. If the mutation status as an analysis target can be determined to be benign, the basic filter 531 sets a rank indicating a benign mutation (e.g., the rank MYC4) and outputs the result to the next filter. If the mutation status as an analysis target cannot be determined to be benign, the basic filter 531 sets a rank indicating a non-benign mutation (e.g., the rank MYC3) and passes the processing to the next filter.

The basic filter 531 receives information from the setting receiving unit 52 to identify the threshold value of the length of the duplication between the base sequence of a known mutation that causes carcinogenesis and the mutant base sequence corresponding to the mutation status, and settings of parameters for each database (compared with the values registered as the benign determination threshold value for determining benignity). The basic filter 531 determines whether the mutation status as an analysis target is benign, based on the settings.

Specifically, if the length of the duplication between the base sequence of a known mutation that causes carcinogenesis and the mutant base sequence corresponding to the mutation status is shorter than the predetermined threshold length value, the basic filter 531 sets a rank indicating a benign mutation. Even in other cases, if the mutation status represents a mutation located in an intron region, the basic filter 531 sets a rank indicating a benign mutation.

Even if the above two criteria are not satisfied, the basic filter 531 searches the specified database, and if the mutation represented by the mutation status is registered in the database, and the registered value indicating the probability of the mutation exceeds the predetermined benign determination threshold value for the database, the basic filter 531 sets a rank indicating a benign mutation.

The time-series filter 532 is the same as the example of the common filter unit 53 in FIG. 5, except that the value subtracted from the rank corresponding to the mutation status as an analysis target differs from the example of the common filter unit 53 in FIG. 5, and that the output destination of the rank calculated by the time-series filter 532 differs from the example of the common filter unit 53 in FIG. 5. The time-series filter 532 references the mutation status information included in the time-series information corresponding to the mutation status as an analysis target, and determines whether the same mutation was present in time-series information extracted at different timings.

The time-series filter 532 uses the mutation status as an analysis target and the corresponding mutation status included in the time-series information. If the same mutation is present, the time-series filter 532 sets a rank indicating potential pathogenicity corresponding to the mutation status as the analysis target (e.g., subtracting the second predetermined amount "2" from the rank), and passes the processing to the quality filter 539. In this example, since the basic filter 531 has passed the processing, the initial rank is MYC3. When the time-series filter 532 determines potential pathogenicity, the rank is set to MYC1 by subtracting the second predetermined amount "2" from MYC3. The second predetermined amount is larger than the first predetermined amount.

On the other hand, the time-series filter 532 uses the mutation status as an analysis target and the corresponding mutation status included in the time-series information. If the same mutation is not present, the time-series filter 532 retains the rank (retaining the initial rank MYC3) and passes the processing to the database filter 533.

The time-series filter 532 may receive settings from the setting receiving unit 52 regarding threshold values for depth, other sequence quality, mutation allele frequency, etc. For example, if the depth related to the corresponding mutation status included in the time-series information does not exceed the set threshold value (e.g., "20"), the time-series filter 532 retains the rank (retaining the initial rank MYC3) and passes the processing to the database filter 533 without determining whether the same mutation status was present.

Similar to the example of the common filter unit 53 in FIG. 5, if the data receiving unit 51 has not received time-series information (i.e., if the mutant base sequence information has been received only as an analysis target), the time-series filter 532 may retain the rank (retaining the initial rank MYC3) and pass the processing to the database filter 533 without determining whether the same mutation status exists.

If the setting receiving unit 52 has input the setting not to use the time-series filter 532, the time-series filter 532 retains the rank (retaining the initial rank MYC3) and passes the processing to the fusion gene filter 536 without determining whether the same mutation status exists.

Hereinafter, a mutated base sequence corresponding to any mutation status included in the mutant base sequence information is also referred to as a mutant base sequence. A fusion gene filter 536 determines whether the mutant base sequence includes a fusion gene, which is formed by the fusion of two genes similar to the two candidate genes of the first fusion genes obtained by the analyzer 1. More specifically, the fusion gene filter 536 determines, for each of the first fusion genes, whether the similarity of the two base sequences encoding the two candidate genes of the first fusion genes obtained by the analyzer 1 and at least part of the base sequence included in the mutant base sequence exceeds a threshold value for both sequences. Similarity is expressed, for example, by the percentage of alignment matches between the two base sequences. If the percentage of alignment matches between the two base sequences exceeds the threshold value, the two base sequences are determined to be similar.

As an example, regarding the BCR-ABL first fusion gene formed by the fusion of the BCR gene and the ABL gene obtained by the analyzer 1, the fusion gene filter 536 calculates the similarity between the base sequence encoding the BCR gene and the corresponding base sequence in the mutant base sequence. Next, the fusion gene filter 536 calculates the similarity between the base sequence encoding the ABL gene in the BCR-ABL first fusion gene and the corresponding base sequence in the mutant base sequence.

The fusion gene filter 536 determines whether both of the calculated similarities exceed the threshold value. The threshold value is, for example, a value assumed to indicate that the activity of the protein encoded by the first fusion gene is similar to the activity of the protein indicated by the mutant base sequence.

If both calculated similarities exceed the threshold value, the fusion gene filter 536 determines that the mutant base sequence includes a fusion gene formed by the fusion of two genes similar to the two candidate genes of the first fusion genes.

On the other hand, if at least one of the calculated similarities is below the threshold value, the fusion gene filter 536 repeats the same determination processing for another first fusion gene obtained by the analyzer 1. If at least one of the calculated similarities is below the threshold value for all the first fusion genes obtained by the analyzer 1, the fusion gene filter 536 determines that the mutant base sequence does not include fusion genes formed by the fusion of two genes similar to the two candidate genes of all the first fusion genes.

If the similarity between the base sequences of the two candidate genes of the first fusion genes obtained by the analyzer 1 and the base sequences of the two genes of the fusion genes included in the mutant base sequence is between 65% and 100% inclusive, the fusion gene filter 536 may determine that the mutant base sequence includes a fusion gene formed by the fusion of two genes similar to the two candidate genes of the first fusion genes. Preferably, if the similarity between the base sequences of the two candidate genes of the first fusion genes and the two genes of the fusion gene included in the mutant base sequence is between 80% and 100% inclusive, the fusion gene filter 536 may determine that the mutant base sequence includes a fusion gene formed by the fusion of two genes similar to the two candidate genes of the first fusion genes.

The fusion gene filter 536 may send the mutant base sequence corresponding to the mutation status as the analysis target to an external server that stores combinations of the candidate genes of the plurality of first fusion genes. The fusion gene filter 536 checks whether the mutant base sequence includes a fusion gene formed by the two genes similar to the two candidate genes of the first fusion genes registered in the database on the external server. If the fusion gene filter 536 receives a notification from the external server indicating that the mutant base sequence includes a fusion gene formed by the two genes similar to the two candidate genes of any of the first fusion genes registered in the database, the fusion gene filter 536 may determine that the mutant base sequence includes a fusion gene formed by the fusion of two genes similar to the two candidate genes of the first fusion genes.

The fusion gene filter 536 determines whether the mutant base sequence includes a fusion gene formed by the fusion of a gene including a base sequence similar to the base sequence of the candidate gene of the second fusion gene obtained by the analyzer 1 and another gene. More specifically, for each of the plurality of second fusion genes obtained by the analyzer 1, the fusion gene filter 536 calculates the similarity between the base sequence of the candidate genes of the second fusion genes and the base sequence of one of the fusion genes included in the mutant base sequence. The fusion gene filter 536 determines whether the calculated similarity exceeds the threshold value. The threshold value is a value assumed to indicate that the activity of the protein encoded by the second fusion gene is similar to the activity of the protein indicated by the mutant base sequence.

If the calculated similarity exceeds the threshold value, the fusion gene filter 536 determines that the mutant base sequence includes fusion genes similar to the candidate genes of the second fusion genes obtained by the analyzer 1. If the calculated similarity is below the threshold value, the fusion gene filter 536 repeats the same determination processing for other candidate genes of the second fusion genes obtained by the analyzer 1. If the calculated similarity is below the threshold value for all the second fusion genes obtained by the analyzer 1, the fusion gene filter 536 determines that the mutant base sequence does not include fusion genes formed by the fusion of genes similar to the candidate genes of the second fusion genes.

If the similarity between the base sequence of the candidate gene of the second fusion genes obtained by the analyzer 1 and the base sequence of one of the fusion genes included in the mutant base sequence is between 65% and 100% inclusive, the fusion gene filter 536 may determine that the mutant base sequence includes a fusion gene formed by the fusion of a gene including the base sequence similar to the base sequence of the candidate gene of the second fusion genes and another gene. Preferably, if the similarity between the base sequence of the candidate gene of the second fusion genes and the base sequences of one of the fusion genes included in the mutant base sequence is between 80% and 100% inclusive, the fusion gene filter 536 may determine that the mutant base sequence includes a fusion gene formed by the fusion of a gene including the base sequence similar to the base sequence of the candidate gene of the second fusion genes and another gene.

The fusion gene filter 536 may send the mutant base sequence to an external server that stores the plurality of second fusion genes. The fusion gene filter 536 checks whether the mutant base sequence includes a fusion gene formed by the fusion of genes similar to any of the candidate genes of the plurality of second fusion genes registered in the database on the external server. If the fusion gene filter 536 receives a notification from the external server indicating that the mutant base sequence includes a fusion gene formed by the fusion of genes similar to any of the candidate genes of the plurality of second fusion genes registered in the database, the fusion gene filter 536 may determine that the mutant base sequence includes a gene similar to the candidate gene of the second fusion genes.

The fusion gene filter 536 determines the rank, based on the result of determining whether the mutant base sequence includes a fusion gene formed by the fusion of two genes similar to the two candidate genes of the first fusion genes. For example, if the fusion gene filter 536 determines that the mutant base sequence includes a fusion gene formed by the fusion of two genes similar to the two candidate genes of any of the plurality of first fusion genes obtained by the analyzer 1, the fusion gene filter 536 determines the rank corresponding to the mutation status as the analysis target indicating potential pathogenicity (for example, by subtracting the second predetermined amount "2" from the rank) and passes the processing to the quality filter 539.

In this manner, the fusion gene filter 536 can accurately estimate the degree of likelihood of pathogenicity of the mutation status, based on the rank by referencing the base sequences of the two candidate genes of the first fusion genes, which are known to likely cause driver mutations.

The fusion gene filter 536 determines the rank, based on the result of determining whether the mutant base sequence includes a fusion gene formed by the fusion of a gene including a base sequence similar to the base sequence of the candidate genes of the second fusion genes and another gene. For example, if the fusion gene filter 536 determines that the mutant base sequence includes a gene similar to any one of the candidate genes of the plurality of second fusion genes obtained by the analyzer 1, the fusion gene filter 536 determines the rank corresponding to the mutation status as the analysis target indicating potential pathogenicity (for example, by subtracting the first predetermined amount "1" from the rank) and passes the processing to the conserved location filter 537.

If the fusion gene filter 536 determines that the mutant base sequence does not include a fusion gene similar to the two candidate genes of the first fusion genes obtained by the analyzer 1, or that the mutant base sequence does not include a fusion gene similar to the candidate genes of the second fusion genes, the fusion gene filter 536 retains the rank (retaining the initial rank MYC3) and passes the processing to the conserved location filter 537.

Even if one of the two candidate genes of the fusion gene is not registered in the storage unit 18, the second fusion genes including specific candidate genes are known to potentially cause driver mutations. The fusion gene filter 536 can accurately present the degree of likelihood of pathogenicity of the mutation status, based on the rank by referencing the base sequences of the candidate genes of the second fusion genes.

Conserved sequences preserved among the genomes of different species often play important roles in the physiological activities of cells. Therefore, if a mutation occurs at the location of a conserved sequence, the mutation status is likely to be pathogenic. The conserved location filter 537 determines the rank, based on whether the mutation site in the mutation status includes the location of the conserved sequence, which is the base sequence preserved among the genomes of different species. More specifically, the conserved location filter 537 determines whether the mutation site includes the location of the conserved sequence, as indicated by the conserved sequence location information obtained by the analyzer 1.

If the conserved location filter 537 determines that the mutation site includes the location of the conserved sequence, the conserved location filter 537 determines the rank corresponding to the mutation status as the analysis target indicating potential pathogenicity (for example, by subtracting the first predetermined amount "1" from the rank) and passes the processing to the structure filter 538. On the other hand, if the conserved location filter 537 determines that the mutation site does not include the location of the conserved sequence, the conserved location filter 537 retains the rank and passes the processing to the structure filter 538. In this manner, the conserved location filter 537 can accurately present the degree of likelihood of pathogenicity of the mutation status corresponding to the mutation site, based on the rank using information indicating the location of the conserved sequence.

It is known that structural variants such as chromosomal translocations, deletions of important genes, and mutations spanning a plurality of genes are likely to be pathogenic. The structure filter determines whether the mutation status indicated by the mutant base sequence information is a structural variant such as a chromosomal translocation.

The structure filter 538 determines whether the mutation status indicated by the mutant base sequence information is a chromosomal translocation, and determines the rank based on the result of determination. The structure filter 538 determines whether a chromosomal translocation has occurred by referring to the details of mutation and the mutation site included in the mutation status indicated by the mutant base sequence information. The structure filter 538 may determine whether the mutation status is a chromosomal translocation by splitting the mutant base sequence corresponding to the mutation status into a plurality of base sequences and identifying the genomic locations for each split base sequence.

The structure filter 538 determines whether the mutation status indicated by the mutant base sequence information is a mutation spanning a plurality of genes, and determines the rank based on the result of determination. The structure filter 538 determines whether a mutation spanning a plurality of genes has occurred by referring to the details of mutation and the mutation site included in any mutation status indicated by the mutant base sequence information. The structure filter 538 may determine whether the mutation status is a mutation spanning a plurality of genes by splitting the mutant base sequence corresponding to the mutation status into a plurality of base sequences and identifying the genomic locations for each split base sequence.

The storage unit 18 is pre-registered with information indicating a plurality of registered genes involved in cellular carcinogenesis. The information indicating the registered genes includes, for example, identification information for identifying the registered genes and information indicating the chromosomal locations of the registered genes. The structure filter 538 may determine whether the mutation status indicated by the mutant base sequence information is a deletion of the registered genes and determine the rank based on the result of determination. The structure filter 538 determines whether any of the plurality of registered genes registered in the storage unit 18 are deleted by referring to the details of mutation and the mutation site included in any mutation status indicated by the mutant base sequence information.

The storage unit 18 is pre-registered with information indicating the chromosomal locations of enhancers that control the expression of genes involved in cellular carcinogenesis. When the structure filter 538 determines that translocations, inversions, or deletions have occurred, the structure filter 538 may determine whether the oncogene, of which the mutation status indicated by the mutant base sequence information is registered in the storage unit 18, is an uncontrolled abnormality located near the enhancer registered in the storage unit 18, and determine the rank based on the result of determination.

The storage unit 18 is pre-registered with information indicating the direction of genomes in the gene regions (5'->3', 3'->5'). When the structure filter 538 determines that the mutation status indicated by the mutant base sequence information forms a fusion gene such as the first fusion gene or the second fusion gene due to translocation or deletion, and when the two genes forming the fusion gene are the first and second candidate genes, the structure filter 538 may determine whether the directions of the first and second candidate genes are the same (for example, the first and second candidate genes are in the 5'->3' and 3'->5' direction, or the first and second candidate genes are in the 3'->5' and 5'->3' direction), determine whether a functional fusion gene is formed, and determine the rank based on the result of determination.

The storage unit 18 is pre-registered with sequence information related to amino acid translation (codons) of gene regions and RNA splicing. When the structure filter 538 determines that the mutation status indicated by the mutant base sequence information forms a fusion gene due to translocation or deletion, the structure filter 538 may use this information to determine whether a functional fusion gene is formed, and determine the rank based on the result of determination.

The structure filter 538 splits the mutant base sequence into a plurality of base sequences, and identifies the genomic locations for each split base sequence. The structure filter 538 may determine whether a deletion has occurred in any of the registered genes by comparing the genomic location of the identified base sequence with the locations of the plurality of registered genes in the storage unit 18.

If the structure filter 538 determines that a translocation has occurred, the structure filter 538 determines the rank corresponding to the mutation status as the analysis target indicating potential pathogenicity. For example, the structure filter 538 subtracts the first predetermined amount "1" from the rank corresponding to the mutation status. On the other hand, if the structure filter 538 determines that a translocation has not occurred, the rank corresponding to the mutation status as the analysis target is retained.

If the structure filter 538 determines that a mutation spanning a plurality of genes has occurred, the structure filter 538 determines the rank corresponding to the mutation status as the analysis target indicating potential pathogenicity (for example, by subtracting the first predetermined amount "1" from the rank corresponding to the mutation status). On the other hand, if the structure filter 538 determines that a structural variant spanning a plurality of genes has not occurred, the structure filter 538 retains the rank corresponding to the mutation status.

If the structure filter 538 determines that a deletion has occurred in any of the plurality of registered genes stored in the storage unit 18, the structure filter 538 further subtracts the first predetermined amount from the rank corresponding to the mutation status as the analysis target, and passes the processing to the quality filter 539. On the other hand, if the structure filter 538 determines that a deletion has not occurred in any of the plurality of registered genes stored in the storage unit 18, the structure filter 538 retains the rank corresponding to the mutation status as the analysis target and passes the processing to the quality filter 539. In this manner, the structure filter 538 can accurately present the degree of likelihood of pathogenicity of the mutation status, based on the rank by determining whether structural variants such as chromosomal translocations, mutations spanning a plurality of genes, and deletions of genes involved in cellular carcinogenesis have occurred.

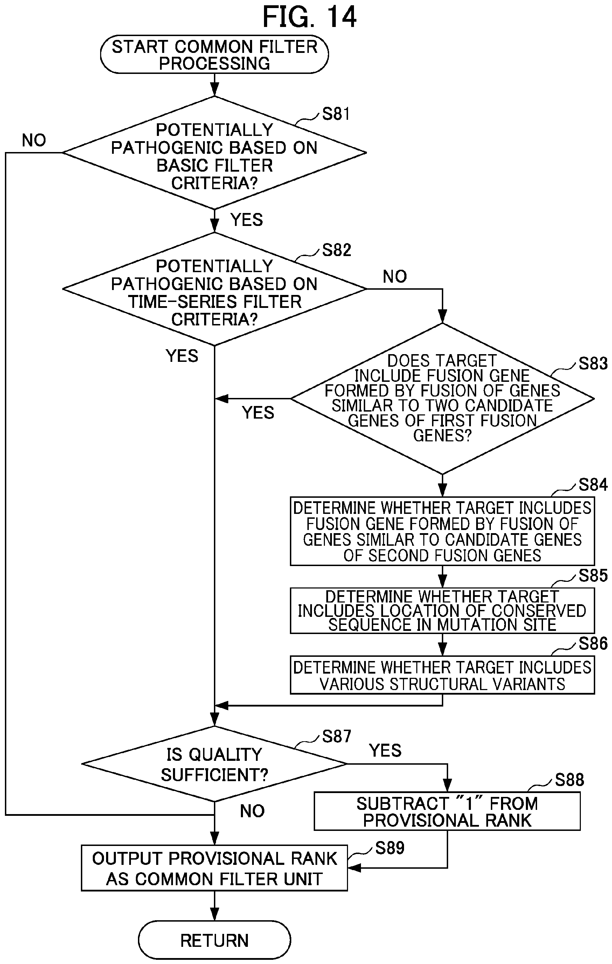

FIG. 14 is a flowchart illustrating the detailed process flow of the common filter processing performed by the common filter unit with the functional configuration illustrated in FIG. 13. In Step S81, the basic filter 531 determines whether the sequence variation data as the processing target is potentially pathogenic based on the basic filter criteria. If the mutation status (sequence variation) as the processing target is determined not to be potentially pathogenic based on the basic filter criteria, the determination in Step S81 is "NO", and the processing proceeds to Step S89. In Step S89, the common filter unit 53 outputs the provisional rank as the common filter unit. Consequently, the common filter processing in Step S3 of FIG. 14 is terminated, and the processing proceeds to Step S4.

If the mutation status (sequence variation) as the processing target is determined to be potentially pathogenic based on the basic filter criteria, the determination in Step S81 is "YES", and the processing proceeds to Step S82.

In Step S82, the time-series filter 532 determines whether the sequence variation data as the processing target is potentially pathogenic based on the time-series filter criteria. If the mutation status (sequence variation) as the processing target is determined to be potentially pathogenic based on the time-series filter criteria, the determination in Step S82 is "YES", and the processing proceeds to Step S87. The processing from Step S87 onward will be described later. If the mutation status (sequence variation) as the processing target is determined not to be potentially pathogenic based on the basic filter criteria, the determination in Step S82 is "NO", and the processing proceeds to Step S83.

In Step S83, the fusion gene filter 536 determines whether the sequence variation data as the processing target includes a fusion gene similar to the two candidate genes of the first fusion genes. If the mutation status (sequence variation) as the processing target includes a fusion gene similar to the two candidate genes of the first fusion genes, the determination in Step S83 is "YES", and the processing proceeds to Step S87. The processing from Step S87 onward will be described later. If the mutation status (sequence variation) as the processing target does not include a fusion gene similar to the two candidate genes of the first fusion genes, the determination in Step S83 is "NO", and the processing proceeds to Step S84.

In Step S84, the fusion gene filter 536 determines whether the sequence variation data as the processing target includes a fusion gene similar to the candidate genes of the second fusion genes.

In Step S85, the conserved location filter 537 determines whether the sequence variation data as the processing target includes the location of the conserved sequence in the mutation site.

In Step S86, the structure filter 538 determines whether the sequence variation data as the processing target includes various structural variants.

In Step S87, the quality filter 539 determines whether the quality is sufficient. If the quality of the results of the processing in Steps S81 to S86 (filter results of the basic filter 531, the time-series filter 532, the fusion gene filter 536, the conserved location filter 537, and the structure filter 538) is sufficient, the determination in Step S87 is "YES", and the processing proceeds to Step S88. Since the quality filter 539 has determined that the quality is sufficient, the first predetermined amount "1" is subtracted from the provisional rank in Step S88.

If the quality of the results of the processing in Steps S81 to S86 (filter results of the basic filter 531, the time-series filter 532, the fusion gene filter 536, the conserved location filter 537, and the structure filter 538) is not sufficient, the determination in Step S87 is "NO", and the processing proceeds to Step S89.

In Step S89, the common filter unit 53 outputs the provisional rank as the common filter unit. Consequently, the common filter processing in Step S3 of FIG. 9 is terminated, and the processing proceeds to Step S4.

Although an embodiment of the present invention has been described above, the present invention is not limited to the aforementioned embodiment, and modifications, improvements, and the like that can achieve the object of the present invention are included in the present invention.

For example, in the above embodiment, the seed gene filter unit 54 and the rescue filter unit 55 are adopted in addition to the common filter unit 53, but this is not particularly limited. In other words, as compared to the case of adopting the common filter unit 53 alone, the filters only need to improve the efficiency and convenience of analyzing the degree of likelihood that the mutation affects the onset or progression of diseases. Such filters may include, for example, the following types of filter units.

Specifically, the following configuration is sufficient for the common filter unit 53. The common filter unit 53 is included in the analyzer 1 that selects the target sequence variations that are present in the subject and that pose a risk of harm. The common filter unit 53 classifies each of the plurality of sequence variations identified by sequencing the nucleic acids contained in the subject, based on the first classification criteria, into a high category that categorizes sequence variations with the highest likelihood of being selected as the target sequence variation (e.g., MYC1) or at least one lower category with a lower likelihood (e.g., MYC2, MYC3, MYC4).

In this case, for example, the structure can adopt a classification criterion setting unit and a second filtering unit as filter units employing rule-based methods as described below, subsequent to the common filter unit 53. The classification criterion setting unit sets a classification criteria of being registered in a database or list, as the second classification criteria (such as the classification criteria of the seed gene filter 541 or the classification criteria of the rescue filter unit 55 employing rule-based methods) different from the first classification criteria for classification into the high category. The second filtering unit reclassifies the sequence variations, which have been classified into the lower category by the common filter unit 53 but satisfy the second classification criteria, into the high category.

For example, the structure can adopt the following second filtering unit employing AI or machine learning methods, subsequent to the common filter unit 53. As a premise, the learning device (not illustrated) executes predetermined machine learning, using a plurality of learning information sets, on predetermined nucleic acids. These learning information sets include information indicating known sequence variations that pose a risk of harm, and clinical significance information on at least some variations from public databases, human genetic polymorphism databases, drug-gene interaction and druggable genome resource databases, and drug response databases. Upon inputting a predetermined sequence variation, the learning device generates or updates a model (such as an AI model) that outputs the degree of likelihood that the predetermined sequence variation is the target sequence variation (e.g., the ranks MYC1 to MYC4). Here, updating means relearning by adding learning information set. The learning device may be provided as part of the analyzer 1 or as a device different from the analyzer 1. In this case, the second filtering unit reclassifies the sequence variations that have been classified into the lower category by the common filter unit 53 and that have been outputted from the model with at least a certain level of likelihood, into the high category.

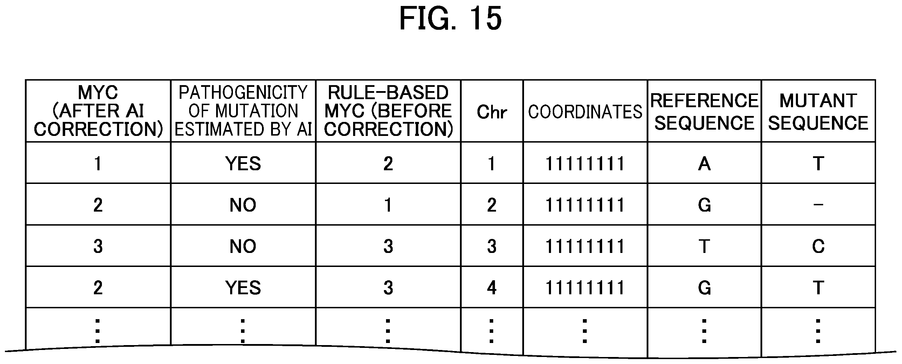

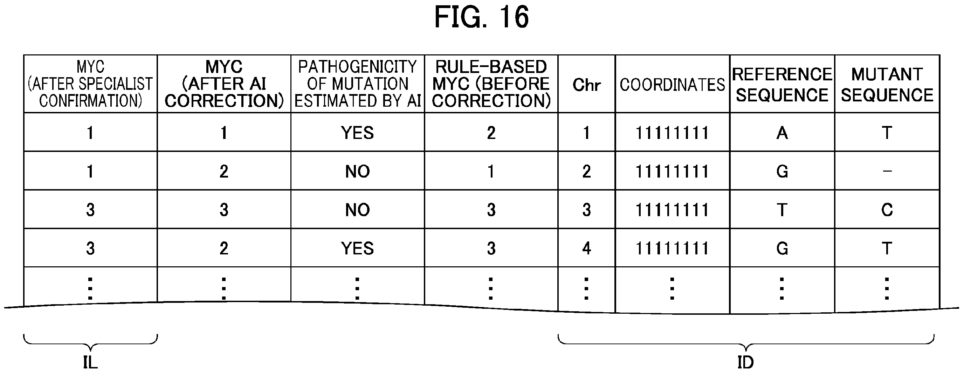

As mentioned above, if the rescue filter unit 55 adopts the classification method using the model (such as an AI model) obtained by machine learning, the rescue filter processing can input the sequence data as the processing target into the model and cause the model to output a higher rank. With reference to FIGS. 15 and 16, the following describes examples of inference using the AI model obtained by machine learning, and generating or updating the AI model.

FIG. 15 is a table illustrating an example of inference using an AI model generated or updated by machine learning in the rescue filter processing of FIG. 12. The inputs and outputs in inference using the AI model include items such as "MYC (after AI correction)", "pathogenicity of the mutation estimated by AI", "rule-based MYC (before correction)", "Chr", "coordinates", "reference sequence", and "mutant sequence" as illustrated in the table in FIG. 15. The "Chr" item refers to the chromosome number where the base sequence of the mutation status (sequence variation) was found, as described above with reference to FIG. 3. The "coordinates" item refers to the coordinates (location) on the reference genome, as described above with reference to FIG. 6. The "reference sequence" item refers to the base sequence at the "coordinates" on the reference genome (a single base in the example in FIG. 15). The "mutant sequence" item refers to the base sequence at the mutation site (coordinates) of the base sequence extracted through sequence alignment from the genetic information of the sample (a single base in the example in FIG. 15).

As mentioned above, this information is input into the rescue filter unit 55, and the sequence variations can be classified using rule-based methods with classification criteria different from those adopted by the common filter unit 53 and the seed gene filter unit 54. Here, the "rule-based MYC (before correction)" item in FIG. 15 is the rank resulting from classification using rule-based methods with classification criteria different from those adopted in the common filter unit 53 and the seed gene filter unit 54 by the rescue filter unit 55.

Furthermore, the rescue filter unit 55 can adopt a method of classification using the model (such as an AI model) obtained by machine learning. The output from the model (such as an AI model) obtained by machine learning adopted by the rescue filter unit 55 can vary, but here "MYC (after AI correction)" as an indicator of whether the mutation is pathogenic is output for correcting the rank. Here, "pathogenicity of the mutation estimated by AI" in FIG. 15 is a model simulating a specialist, and indicates whether the mutation is pathogenic, based on a model (such as an AI model) obtained by machine learning. For example, a model is generated based on a rule-based method (e.g., a method using the judgment criteria of specialists), which estimates and outputs whether the mutation is pathogenic, based on inputs of information such as "Chr", "coordinates", "reference sequence", and "mutant sequence". As a result, for the mutations determined to involve "pathogenicity of the mutation estimated by AI", the rescue filter unit 55 outputs "MYC (after AI correction)" by subtracting the predetermined amount "1" from the "rule-based MYC (before correction)". In this manner, the rescue filter unit 55 can output "MYC (after AI correction)" as an inference result using the classification method with the model (such as an AI model) obtained by machine learning. For example, the model (such as an AI model) obtained by machine learning adopted by the rescue filter unit 55 may be trained to output "MYC (after AI correction)" instead of outputting "pathogenicity of the mutation estimated by AI".

FIG. 16 is a table illustrating an example of updating an AI model generated or updated by machine learning in the rescue filter processing of FIG. 12. Specifically, the "MYC (after specialist confirmation)" item in FIG. 16 represents the MYC rank after being verified by specialists who have reflected their judgments or detailed examinations on the various information included in FIG. 15. In the fourth row (fifth row including the item names) of the table illustrated in FIG. 16, the "MYC (after specialist confirmation)" differs from the "MYC (after AI correction)". Therefore, the model (such as an AI model) obtained by machine learning can learn (can be updated) to output the output information IL when the input information ID is input. Thus, the rescue filter unit 55 can be trained to reproduce the "MYC (after specialist confirmation)" using the AI model obtained by machine learning. In other words, this improves the accuracy of the rescue filter unit 55, which adopts the AI model obtained by machine learning. Consequently, the accuracy of the rescue filter processing in FIG. 12 can be enhanced by utilizing the AI model obtained by machine learning.

As described above, accuracy can be improved by using inference with an AI model generated or updated by machine learning in the rescue filter unit 55. The following further describes an example of using an AI model generated or updated by machine learning in the seed gene filter processing in the seed gene filter unit 54.

Specifically, the AI model generated or updated by machine learning may be used in the seed gene filter processing. For example, the model (such as an AI model) can be generated by learning to propose correction values to optimize the threshold values (cutoff values) or parameters used in the seed gene filter processing, based on the clinical information and the MYC ranks confirmed by specialists.

The model (such as an AI model) can use the clinical information including the provisional ranks obtained by the common filter unit 53 and the seed gene information acquired by the seed gene information acquisition unit 543, at least as part of the learning data. The model (such as an AI model) can use the "MYC (after specialist confirmation)" information in FIG. 16, at least as part of the learning data. As a result, the model (such as an AI model) can output correction values to optimize the threshold values (cutoff values) or parameters used in the seed gene filter processing. Users such as specialists review the correction values proposed by the model (such as an AI model) and determine the values to actually use as threshold values (cutoff values) or parameters in the seed gene filter processing. In other words, users such as specialists consider the correction values proposed by the model (such as an AI model), and for example, determine the parameters to input into the screen example in FIG. 8. Thus, the proposal by the model (such as an AI model) allows the users such as specialists to adopt threshold values (cutoff values) or parameters considered more suitable for the seed gene filter processing.

This results in the implementation of a hybrid AI that combines the benefits of the rule-based AI familiar to specialists and the machine learning. In other words, the assignment of the MYC rank in the seed gene filter processing is carried out based on rules, making the parameters explainable. Then, the model (such as an AI model) corrects the parameters. Traditionally, processing using AI models have often been black-box operations, lacking transparency regarding the basis for processing (such as filtering processing). However, the described model (such as an AI model) can address this by outputting correction values to optimize explainable threshold values (cutoff values) or parameters. Based on this, it is possible to achieve improvements in the efficiency of interpretation through rule-based filtering that ensures explainability similar to human methods, and enhancements in filtering accuracy through the improvement of rules (features) by models (such as AI models).

The system configuration illustrated in FIG. 4 and the hardware configuration of the analyzer 1 illustrated in FIG. 5 are merely examples to achieve the objects of the present invention and are not particularly limited.

The functional block diagram illustrated in FIG. 6 is also an example and is not particularly limited. That is, as long as the information processing system is provided with the functionality to execute the series of processing described above, the types of functional blocks used to achieve this functionality are not limited to the example in FIG. 6.

The locations of the functional blocks are not limited to those illustrated in FIG. 6 and can be arbitrary. For example, in the example of FIG. 6, the processing is performed on the analyzer 1 side, but this is not limited to this configuration, and at least part of the processing may be performed on another information processing device (not illustrated). That is, the functional blocks required to execute the analysis processing are configured on the analyzer 1 side, but this is merely an example. At least part of the functional blocks arranged on the analyzer 1 side may be configured on another information processing device side (not illustrated).

The series of processing described above can be executed by hardware or by software. A single functional block may be composed solely of hardware, solely of software, or a combination of both.

When executing the series of processing by software, the program constituting the software is installed on a computer from a network or a recording medium. The computer may be a computer embedded in dedicated hardware. The computer may also be a general-purpose computer, such as a server, a smartphone, or a personal computer, capable of executing various functions by installing various programs.

The recording medium containing such a program is not limited to removable medium (not illustrated) distributed separately from the main device, but can also be a recording medium provided in a state pre-installed in the main device.

In this specification, the steps describing the program recorded on a recording medium include not only processing performed chronologically in sequence but also processing that can be executed in parallel or individually without necessarily following the chronological order. In this specification, the term "system" refers to an overall apparatus composed of a plurality of devices or a plurality of means.

In summary, the information processing system to which the present invention is applied can take the following configuration, accommodating various embodiments.

That is, the information processing device to which the present invention is applied is:
an information processing device (e.g., analyzer 1 in FIG. 2) that selects target sequence variations that are present in a subject and that pose a risk of harm (e.g., cancer driver mutations), in which the information processing device only needs to include:
a first filterer (e.g., the common filter unit 53 in FIG. 2 or 5) that classifies each of the plurality of sequence variations identified by sequencing the nucleic acid contained in the subject, based on a first classification criterion, into either a high category (for example, rank MYC1 in the specification) that categorizes sequence variations with the highest likelihood of being selected as the target sequence variations, or at least one lower category (for example, the ranks MYC2 to MYC4 in the specification) with a lower likelihood;
a classification criterion setter (e.g., the parameter setting receiving unit 542 in FIG. 2 of the seed gene filter unit 54 in FIG. 6, and part of the rescue filter unit 55 employing rule-based methods) that sets a classification criterion of being registered in a database or list, as a second classification criterion different from the first classification criterion for classification into the high category; and
a second filterer (e.g., seed gene filter 541 in FIG. 6 of the seed gene filter unit 54 in FIG. 2, and the rescue filter of the rescue filter unit 55 employing rule-based methods) that reclassifies the sequence variations, which have been classified into the lower category by the first filterer but satisfy the second classification criterion, into the high category. Thus, among the results of filtering by the first filterer, the sequence variations, which have been classified into the high category with the highest likelihood of being selected as the target sequence variations but should have been classified into the lower category, are reclassified into the lower category, and the sequence variations, which have been classified into the category with lower likelihoods of being selected as the target sequence variations but should have been classified into the high category, are reclassified into the high category. Consequently, this improves the efficiency and convenience of analyzing the degree of likelihood that the mutation affects the onset or progression of diseases.

Furthermore, the classification criterion setter can: input the minimum number of registrations in the database, as a parameter for setting the second classification criterion (e.g., the cutoff value for the number of registered samples in COSMIC to be inputted in the designated field A1 of FIG. 8), and
set the classification criterion of being registered in the database with at least the minimum number of registrations, as the second classification criterion.

Moreover, the classification criterion setter can: input a specific database or specific list (e.g., the database or guideline containing weighted genes to be inputted in the designated field A3 or the region RS in FIG. 8), as a parameter for setting the second classification criterion, and set the classification criterion of being registered in the specific database or specific list, as the second classification criterion.

Additionally, the classification criterion setter can: input a predetermined disease (e.g., the carcinoma type specified by the user in the designated field A2 of FIG. 8), as a parameter for setting the second classification criterion, and
set at least one of the classification criterion of being registered in the database or list for the predetermined disease, and the classification criterion of being registered in the database or list as a sequence variation for the predetermined disease, as the second classification criterion.

Furthermore, the classification criterion setter can: input information indicating a specific nucleic acid or a sequence of the specific nucleic acid (e.g., location information on the user-specified weighted sequence or the user-specified specific sequence to be inputted in the designated field A4 of FIG. 8 (such as genes, microRNAs, untranslated regions, regulatory regions such as transcription regulatory elements such as promoters and enhancers; in particular, hg19 or GRCh38/hg38 genomic coordinates in humans)), as a parameter for setting the second classification criterion, and
set the classification criterion of corresponding to the sequence variation of the specific nucleic acid or being registered in the database or list, as the second classification criterion.

Additionally, the second filterer can reclassify (e.g., "downgrade" as mentioned in the specification) the sequence variations, which have been classified into the high category by the first filterer but do not satisfy the second classification criterion, into the lower category.

The information processing system to which the present invention is applied is:
an information processing system (the information processing system including the analyzer 1 in FIG. 2) that selects target sequence variations that are present in a subject and that pose a risk of harm, in which the information processing system only needs to include:
a learner that executes predetermined machine learning, using a plurality of learning information sets, on predetermined nucleic acids. These learning information sets include information indicating known sequence variations that pose a risk of harm, and clinical significance information on at least some variations from a public database, a human genetic polymorphism database, a drug-gene interaction and druggable genome resource database, or a drug response database, and upon inputting a predetermined sequence variation, generates or updates a model (such as an AI model) that outputs the degree of likelihood that the predetermined sequence variation is the target sequence variation;
a first filterer (e.g., the common filter unit 53 in FIG. 2 or 5) that classifies each of the plurality of sequence variations identified by sequencing the nucleic acid contained in the subject, based on a predetermined classification criterion, into either a high category (for example, rank MYC1) that categorizes sequence variations with the highest likelihood of being selected as the target sequence variation, or at least one lower category (for example, the ranks MYC2, MYC3, and MYC4) with a lower likelihood than the high category; and
a second filterer (e.g., the rescue filter unit 55 in FIG. 2, to which the machine learning techniques such as AI is applied) that reclassifies the sequence variations that have been classified into the lower category by the first filterer and that have been outputted from the model with at least a certain level of likelihood, into the high category.

For example, public databases that can be adopted include ClinVar (a database for human genome variations and related diseases and genetic disorders) and the aforementioned COSMIC. For example, the dbsnp can be adopted as a database for human genetic polymorphisms. For example, the DGId can be adopted as a database for drug-gene interactions and druggable genome resources. For example, PharmGKB or OncoKB can be adopted as a database for drug response.

Furthermore, the present invention is applied to an information processing device (e.g., analyzer 1 in FIG. 2) that selects target sequence variations that are present in a subject and that pose a risk of harm, in a case where a predetermined storage medium stores a model that is obtained by executing predetermined machine learning, using a plurality of learning information sets, on predetermined nucleic acids, the learning information sets including information indicating known sequence variations that pose a risk of harm, and clinical significance information on at least some variations from a public database, a human genetic polymorphism database, a drug-gene interaction and druggable genome resource database, and a drug response database, and upon inputting a predetermined sequence variation, the model outputting the degree of likelihood that the predetermined sequence variation is the target sequence variation, in which the information processing device can include:
a first filterer (e.g., the common filter unit 53 in FIG. 2 or 5) that classifies each of the plurality of sequence variations identified by sequencing the nucleic acid contained in the subject, based on a predetermined classification criterion, into either a high category that categorizes sequence variations with the highest likelihood of being selected as the target sequence variation, or at least one lower category with a lower likelihood than the high category; and
a second filterer (e.g., the rescue filter unit 55 in FIG. 2, to which the machine learning techniques such as AI is applied) that reclassifies the sequence variations that have been classified into the lower category by the first filterer and that have been outputted from the model with at least a certain level of likelihood, into the high category.

### EXPLANATION OF REFERENCE NUMERALS

- 1:: analyzer
- 11:: CPU
- 18:: storage unit
- 20:: drive
- 31:: removable medium
- 51:: data receiving unit
- 52:: setting receiving unit
- 53:: common filter unit
- 54:: seed gene filter unit
- 55:: rescue filter unit
- 56:: rank determination unit
- 57:: analysis result output unit
- 531:: basic filter
- 532:: time-series filter
- 533:: database filter
- 534:: functional prediction filter
- 535:: quality filter
- 541:: seed gene filter
- 542:: parameter setting receiving unit
- 543:: seed gene information acquisition unit

## Claims

1. An information processing device that selects target sequence variations that are present in a subject and that pose a risk of harm, the device comprising:
a first filterer that classifies each of a plurality of sequence variations identified by sequencing nucleic acids contained in the subject, based on a first classification criterion, into either a high category that categorizes sequence variations with a highest likelihood of being selected as the target sequence variations or at least one lower category with a lower likelihood;
a classification criterion setter that sets a classification criterion of being registered in a database or list, as a second classification criterion different from the first classification criterion for classification into the high category; and
a second filterer that reclassifies the sequence variations, which have been classified into the lower category by the first filterer but satisfy the second classification criterion, into the high category.

2. The information processing device according to claim 1, wherein
the classification criterion setter
inputs a minimum number of registrations in the database, as a parameter for setting the second classification criterion, and
sets a classification criterion of being registered in the database with at least the minimum number of registrations, as the second classification criterion.

3. The information processing device according to claim 1 or 2, wherein
the classification criterion setter
inputs a specific database or specific list, as a parameter for setting the second classification criterion, and
sets a classification criterion of being registered in the specific database or specific list, as the second classification criterion.

4. The information processing device according to any one of claims 1 to 3, wherein
the classification criterion setter
inputs a predetermined disease, as a parameter for setting the second classification criterion, and
sets at least one of a classification criterion of being registered in a database or list related to the predetermined disease or a classification criterion of being registered as a sequence variation related to the predetermined disease in the database or list, as the second classification criterion.

5. The information processing device according to any one of claims 1 to 4, wherein
the classification criterion setter
inputs information indicating a specific nucleic acid or a sequence of the specific nucleic acid, as a parameter for setting the second classification criterion, and
sets a classification criterion corresponding to a sequence variation of the specific nucleic acid or being registered in the database or list, as the second classification criterion.

6. The information processing device according to any one of claims 1 to 5, wherein
the second filterer further reclassifies the sequence variations, which have been classified into the high category by the first filterer but do not satisfy the second classification criterion, into the lower category.

7. An information processing method executed by an information processing device that selects target sequence variations that are present in a subject and that pose a risk of harm, the method comprising:
a first filtering step of classifying each of a plurality of sequence variations identified by sequencing nucleic acids contained in the subject, based on a first classification criterion, into either a high category that categorizes sequence variations with a highest likelihood of being selected as the target sequence variations or at least one lower category with a lower likelihood;
a classification criterion setting step of setting a classification criterion of being registered in a database or list, as a second classification criterion different from the first classification criterion for classification into the high category; and
a second filtering step of reclassifying the sequence variations, which have been classified into the lower category by processing in the first filtering step but satisfy the second classification criterion, into the high category.

8. A program for causing a computer that selects target sequence variations that are present in a subject and that pose a risk of harm to execute control processing comprising:
a first filtering step of classifying each of a plurality of sequence variations identified by sequencing nucleic acids contained in the subject, based on a first classification criterion, into either a high category that categorizes sequence variations with a highest likelihood of being selected as the target sequence variations or at least one lower category with a lower likelihood;
a classification criterion setting step of setting a classification criterion of being registered in a database or list, as a second classification criterion different from the first classification criterion for classification into the high category; and
a second filtering step of reclassifying the sequence variations, which have been classified into the lower category by processing in the first filtering step but satisfy the second classification criterion, into the high category.
